(19) Europäisches Patentamt
European Patent Office
Office européen des brevets

(11) Publication number: **0 343 643 B1**

(12) # EUROPEAN PATENT SPECIFICATION

(45) Date of publication of patent specification: **23.03.94**

(51) Int. Cl.5: **A61K 31/41**, A61K 31/415, A61K 31/42, A61K 31/425, A61K 31/33, A61K 45/06, C07D 233/66, C07D 277/32, C07D 263/34

(21) Application number: **89109406.2**

(22) Date of filing: **24.05.89**

The file contains technical information submitted after the application was filed and not included in this specification

(54) **Arylmethylenyl derivatives of thiazolidnones, imidazolidinones and oxazolidinones useful as antiallergy agents and antiinflamatory agents.**

(30) Priority: **25.05.88 US 198528**
**10.04.89 US 334346**

(43) Date of publication of application:
**29.11.89 Bulletin 89/48**

(45) Publication of the grant of the patent:
**23.03.94 Bulletin 94/12**

(84) Designated Contracting States:
**AT BE CH DE ES FR GB GR IT LI LU NL SE**

(56) References cited:
**EP-A- 0 211 670**
**DE-A- 3 023 349**

**J. PHARM. SOC. JAP., vol. 76, 1956 H.TANIYAMA et al. "Studies on Chemotherapeutics for Mycobacterium tuberculosis. XI. Synthesis and Antibac- terial Activity of 4-Thiazo- lidine-Derivatives containing the alpha,beta-Un- saturated Ketone Group (1)" pages 154-157**

(73) Proprietor: **WARNER-LAMBERT COMPANY**
**201 Tabor Road**
**Morris Plains New Jersey 07950(US)**

(72) Inventor: **Cetenko, Wiaczeslaw Antin**
**3565 Green Brier Road**
**Ann Arbor Michigan 48105(US)**
Inventor: **Connor, David Thomas**
**2453 Antietam**
**Ann Arbor Michigan 48105(US)**
Inventor: **Sorenson, Roderick Joseph**
**2820 Briarcliff**
**Ann Arbor Michigan 48105(US)**
Inventor: **Unangst, Paul Charles**
**3659 Middleton Drive**
**Ann Arbor Michigan 48105(US)**
Inventor: **Stabler, Stephen Russell**
**2211 Latham Street No. 304**
**Mountainview California 94040(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 343 643 B1

**EGYPTIAN JOURNAL OF CHEMISTRY, vol. 26, 1983, The Egyptian Chemical Society H.H.MOHARRAM et al. "Syn- thesis of some New Rhodanine Derivatives of Antibacterial and Anticancer Effects" pages 301-311**

**CHEMICAL & PHARMACEUTICAL BULLETIN, vol. 34, 1986, Pharmaceutical Society of Japan J.KATSUMI et al. "Studies on Styrene Derivatives. II. Synthesis and Antiinflammatory Activity of 3,5-Di-tert- -butyl-4-hydroxystyrenes" pages 1619-1627**

**INTERNATIONAL JOURNAL OF SULFUR CHEMISTRY, A-vol. 2, no. 1, 1972, The Intra- -Science Research Foundation T.R.BOSIN et al. "A Novel Product From Oxidation of beta- (3-Malogenophenyl)- -alpha-Mercaptoacrylic Acids" pages 261-266**

**FARMATSEVTICHNIJ ZHURUAL, vol. 25, no. 6, 1970, Kiev N.E.PLEVACHUK "Synthesis and transformation of 3-alkyl (aryl)-4-thionothiazoli- dones" pages 3-6**

**YAKUGAKU ZASSHI, vol. 92, 1972, Pharmaceutical Society of Japan M.GOSHA et al. "Antimicrobial Activity of 5-Oxothiazolidine-2-thione and Rhodanine Derivatives" pages 490-497**

**PATENT ABSTRACTS OF JAPAN, unexamined applications, section C, vol. 10, no. 156, June 5, 1986 THE PATENT OFFICE JAPANESE GOVERNMENT page 126 C 351**

**ARZNEIMITTEL-FORSCHUNG, vol. 19, 1969, Editir Cantor KG T.ZSOLNAI "Die antimikrobielle Wirkung von potentiellen Isothiocyanat- -Bildnern" pages 558-572**

**ACTA PHYTOPATHOLOGICA ACADEMIAE SCIENTARUM HUNGARICAE, vol. 4, 1969, Akademiai Kiado, Budapest G.MATOLCSY et al. "Studies on the Antimicrobial Action of Compounds Containing 1-Oxo-2-methylene- ,1,2-Dioxo- and Derived Groupings" pages 345-351**

**CHEMICAL REVIEWS, vol. 61, 1961, American Chemical Society F.C.BROWN "4-Thiazolidinones" pages 463-521**

**JOURNAL OF THE ORGANIC CHEMISTRY OF THE USSR A TRANSLATION OF ZHURNAL ORGANICHESKOI KHIMII, vol. 14, no. 5, part 1, 1978 N.A.SHENBERG et al. "In- vestigation of the Reactivity and Tantomerism of Azolidines. XXIII. Allytation of Salls of 5-Benzylidene-2- -Thiooxazolidin-4-ones" pages 1225-1228**

(74) Representative: **Mansmann, Ivo**
**Gödecke AG**
**Patentwesen**
**Mooswaldallee 1-9**
**D-79090 Freiburg (DE)**

## Description

It has now been found that compounds selected from novel arylmethylenyl derivatives of thiazolidinones and oxazolidinones have activity useful for treating allergies or inflammation.

Thus, the present invention is for compounds of the formula (I)

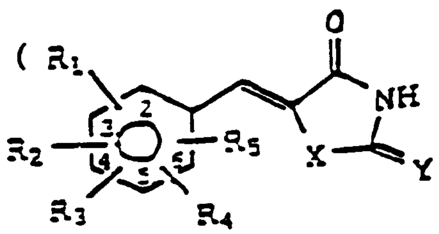

I

and pharmaceutically acceptable salts thereof wherein X and Y are sulfur or oxygen and wherein $R_1$, $R_2$, $R_3$, $R_4$ and $R_5$ are hydrogen or from one to three of $R_1$, $R_2$, $R_3$, $R_4$ and $R_5$ are alkyl or alkoxy of 1 to 6 carbon atoms, hydroxy, halogen, trifluoromethyl, $NO_2$, mercapto, or alkylthio of 1 to 6 carbon atoms or $NR_{10}R_{11}$ wherein $R_{10}$ and $R_{11}$ are independently hydrogen or alkyl of 1 to 6 carbon atoms, however with

proviso 1.) that X and Y may not both be sulfur,

proviso 2.) that when X is sulfur (and Y is oxygen)

a) $R_1$, $R_2$, $R_3$, $R_4$ and $R_5$ may not simultaneously be hydrogen,

b) in case of a monosubstitution of the phenyl ring $R_1$, $R_2$, $R_3$ $R_4$ or $R_5$ is not halogen, hydroxy, nitro, $NR_{10}R_{11}$ or methoxy and

c) in case of a disubstitution of the phenyl ring $R_1$, $R_2$, $R_3$, $R_4$ and $R_5$ is not hydroxy when the second substituent is halogen or hydroxy.

Japanese Application No. 84-133826 as found in Derwent Abstracts No. 87-076379/11 discloses new 3,5-diisopropylbenzylidene-2,4-imidazolidinone and the analogous thiazoles with antiallergic and tyrosine kinase inhibiting activity as well as other heterocyclic containing compounds. EP No. 211,670A as described in Derwent 87-051809/08 describes compounds excluded by the proviso above having a 3,5-diisobutylbenzylidene substituent useful for treating inflammation, ischaemia induced cell damage and arthritis. Thus, known heterocyclic compounds are not as closely related as these named here and are excluded from the present invention.

French Patent No. 2,169,334, describes a series of N-substituted oxazoles as antiarthritics, antirheumatics, and immunosuppressants having the formula

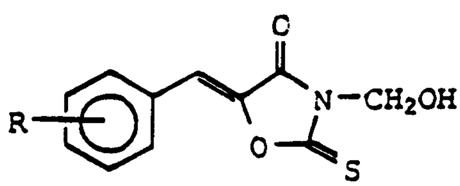

which are not included in the present invention.

The compounds of the formula I have a hitherto unknown pharmaceutical activity useful for treating allergic or inflammatory conditions or diseases. The invention compounds have now also been found to possess an activity as inhibitors of 5-lipoxygenase and/or cyclooxygenase providing treatment of conditions advantageously affected by such inhibition including inflammation, arthritis, pain and pyrrhia.

Thus, the present invention also includes a pharmaceutical composition for diseases or conditions advantageously affected by activity inhibiting singly or together 5-lipoxygenase and cyclooxygenase.

Therefore, the present invention relates particularly to a pharmaceutical composition useful for the treatment of allergy or inflammation which comprises a compound of the formula I together with a pharmaceutically acceptable carrier. Further, the present invention comprises the use of the above compounds for the preparation of pharmaceuticals for treating allergies or inflammation in subject suffering therefrom.

A preferred embodiment of the formula I of the present invention is the composition or the use of a compound of the formula (II)

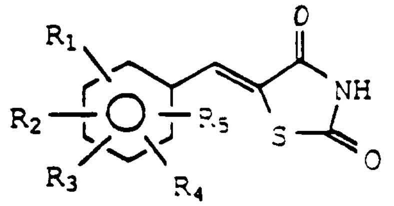

. II

or pharmaceutically acceptable salts thereof; wherein $R_1$, $R_2$, $R_3$, $R_4$ and $R_5$ are as defined above.

Also among the preferred embodiment of the formula I is the composition and use of the compound of the formula (III)

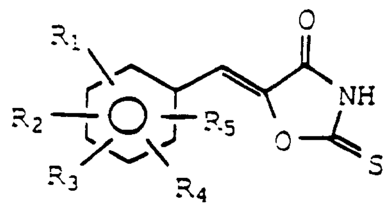

III

or pharmaceutically acceptable salts thereof; wherein $R_1$, $R_2$, $R_3$, $R_4$ and $R_5$ are as defined above.
And finally, a preferred embodiment of the formula I is the composition and the use of the compounds of the formula IV

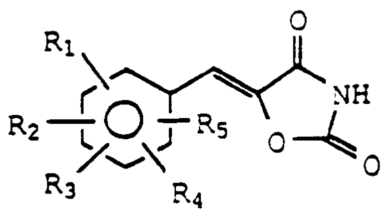

IV

or pharmaceutically acceptable salts thereof; wherein $R_1$, $R_2$, $R_3$, $R_4$ and $R_5$ are as defined above.

The most preferred embodiment of the formula I is the composition or the use of the following compounds which inhibit the release of histamine from human basophils as described hereinafter in the HHB assay stimulated with anti IgE at an $IC_{50}$ less than $\mu$M.

5-[(4-hydroxy-3,5-dimethoxyphenyl)methylene]2,4-thiazolidinedione,
5-[(4-hydroxy-3-methoxyphenyl)methylene]-2,4-thiazolidinedione,
5-[(4-hydroxy-3-methoxyphenyl)methylene]-2-thioxo-4-oxazolidinone,
5-[(4-hydroxy-3,5-dimethoxyphenyl)methylene]-2-thioxo-4-oxazolidinone,
2-thioxo-5-[(3,4,5-trimethoxyphenyl)methylene]-4-oxazolidinone,

Novel compounds which inhibit singly or together 5-lipoxygenase and cyclooxygenase are found by determining percent inhibition in ARBL/ARBC Whole Cell 5-lipoxygenase and Cyclooxygenase Assays and Carrageenan-Induced Rat Foot Paw Edema-2 (CFE-2) Assay described hereinafter.

Among the preferred compounds as determined in the ARBL/ARBC and CFE-2 assays are the following:

2,4-thiazolidinedione, 5-[[3,5-bis(1,1-dimethylethyl)-4-hydroxyphenyl]methylene]-,(E)-
4-oxazolidinone, 5-[[3,5-bis(1,1-dimethylethyl)-4-hydroxyphenyl]methylene]-2-thioxo
2,4-oxazolidinedione, 5-[[3,5-bis(1,1-dimethylethyl)-4-hydroxyphenyl]methylene]-,
2,4-oxazolidinedione, 5-[(4-hydroxy-3,5-dimethoxyphenyl)methylene]-.

The most preferred novel compounds of the present invention now found having activity which inhibit 5-lipoxygenase and cyclooxygenase are 5-[[3,5-bis(1,1-dimethylethyl)-4-hydroxyphenyl]methylene]-2,4-thiazolidinedione and 5-[(3,5-bis(1,1-dimethylethyl)-4-hydroxyphenyl]methylene-2-thioxo-4-oxazolidinone.

Thus, the present invention is also a pharmaceutical composition useful for the treatment of conditions advantageously affected by the inhibition of 5-lipoxygenase and/or cyclooxygenase which comprises a compound of the formula I or various preferred embodiments and the pharmaceutically acceptable acid addition or base salt thereof together with a pharmaceutically acceptable carrier.

The invention also provides for use of any such compound of formula I or particularly various preferred embodiments or salt thereof in the manufacture of medical therapeutic agents for treating allergies and inflammations.

Pharmaceutical composition or use of the compound or salt of formula I is meant to include treatment understood to be prophylactic pertinent to the foregoing named condition.

In the present invention "lower alkyl" is alkyl of from one to six carbons, inclusive, and means methyl, ethyl, propyl, butyl, pentyl, or hexyl and isomers thereof.

"Lower alkoxy" means methoxy, ethoxy, propoxy, butoxy, pentoxy, or hexoxy and isomers thereof.

"Lower alkylthio" means methylmercapto, ethylmercapto, propylmercapto, butylmercapto, pentylmercapto, or hexylmercapto and isomers thereof.

"Halogen" is chloro, bromo, fluoro, or iodo.

Generally, the selected novel compounds of formula I as well as the known compounds are prepared by processes that are known or are prepared by processes analogous to those that are known from known starting materials or starting materials that can be prepared by known methods. For example, the following starting materials are obtained as follows:

3-Bromo-4-hydroxybenzaldehyde and 3,5-dibromo-4-hydroxybenzaldehydeare prepared according to Paal, Chem. Ber. 28 (1895), 2407.

4,5-Dimethoxy-2-hydroxybenzaldehyde is prepared according to Robinson & Head, J. Chem. Soc. -(1930), 2440.

3-Methoxy-5-hydroxybenzaldehyde is prepared according to Ben, et al, J. Org. Chem. 50 (1985), 2238.

3,5-Diisopropyl-4-hydroxybenzaldehyde and 3,5-dimethyl 4-hydroxybenzaldehyde are prepared according to U.S. Patent No. 4,009,210.

3-(dimethylamino)methyl-4-hydroxy-5-methoxybenzaldehyde is prepared according to Hemetsberger, Monats. Chem. 102 (1971), 1110.

3-Methylhydantoin is prepared according to Güler and Moodie, J. Chem. Soc. Perk II (1980), 1752.

A scheme for preparation of the compounds of formula I above is as follows:

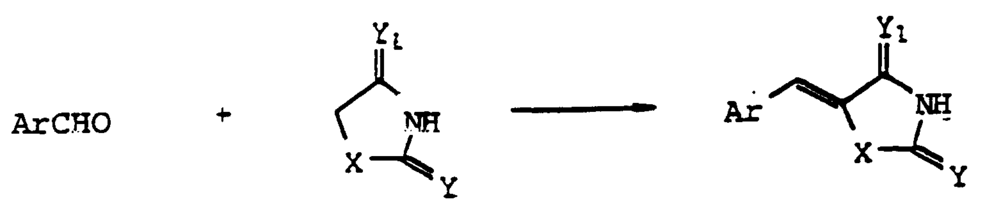

2 3 1

Scheme I

wherein Ar is

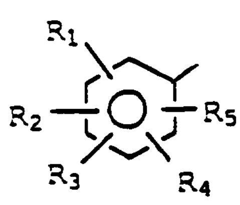

and $R_1$, $R_2$, $R_3$, $R_4$, $R_5$ and X and Y are as defined above.

More particularly, the present invention includes compounds of type 1:

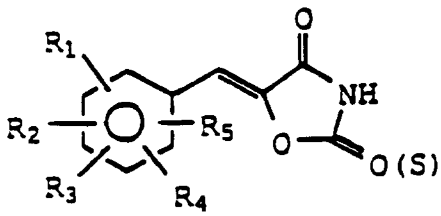

1

where $R_1$, $R_2$, $R_3$, $R_4$ and $R_5$ are as defined above. These compounds are named as substituted 5-phenylmethylene-2-thioxo-4-oxazolidinones, or 5-phenylmethylene-2,4-oxazolidinediones.

Salts of these compounds can be formed by treatment with organic and inorganic bases due to the presence of an acidic NH.

Certain thiazolidinones and oxazolidinones are acidic (pKa ~ 3-6) due to the presence of a tautomeric proton on the heterocyclic ring:

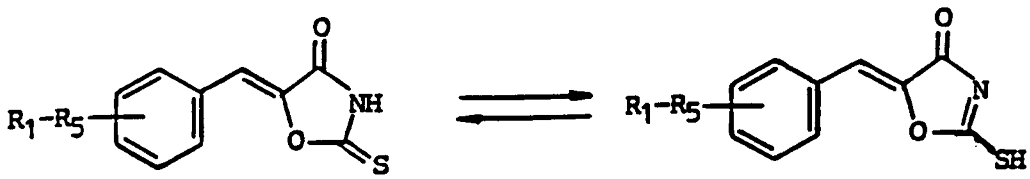

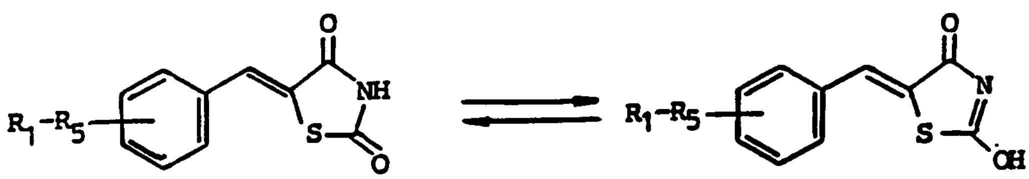

These compounds can form salts with inorganic and organic bases, including choline derivatives.

If the parent oxazolidinone or thiazolidinone and choline free base or a choline derivative (such as choline bicarbonate or choline chloride) are combined in a suitable solvent (such as an alcohol, water, or an alcohol/water solution), the choline salt can generally be obtained by precipitation from solution or by evaporation of the reaction solution.

A general method of preparation of these compounds is the aldol condensation of an aldehyde 2 with an active methylene compound 3 (Scheme 1 above). This condensation can be carried out in alcoholic solvents in the presence of a base such as ammonia, ammonium salts, or piperidine, or with mineral acid

Scheme 1'

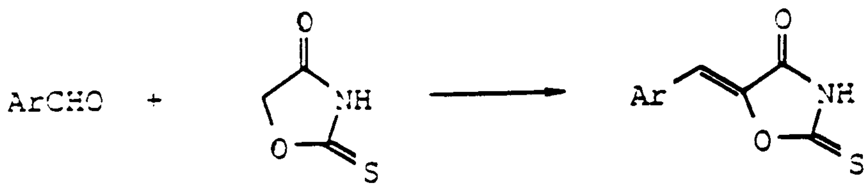

2

I wherein X is O
Y is S and
Ar is as defined
above.

catalysis. A particularly favored procedure is the use of anhydrous sodium acetate in glacial acetic acid, with heating at reflux for 1-24 hours. References to this procedure include: G. R. Newkome and A. Nayak, in Advances in Heterocyclic Chemistry, A. R. Katritzky and A. J. Boulton, Eds., Academic Press, New York, NY, Vol. 25, p. 83 and N. K. Ushenko and T. E. Gorizdra, Ukrain Khim. Zhur., 16, 545 (1950).

An alternate procedure for the preparation of the above compound when Y = O is the preparation of the corresponding oxazole 4 followed by oxidation/hydrolysis to the desired oxazole I wherein X is O and Y is 0 (Scheme 2):

Scheme 2

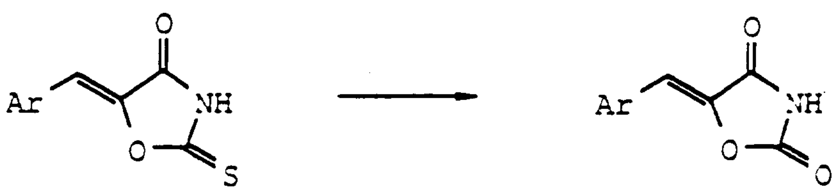

4

I wherein X is O
and
Y is O.

For the preparation of 4, see, for example, F. C. Brown, Chem. Rev., 61, 463 (1961), and the (Newkome) reference previously cited. For the conversion 4 to I of Scheme 2, see also J. W. Clark-Lewis, Chem. Rev., 58, 63 (1958), and N. A. Shenberg, L. S. Guseva, A. I. Ginak, and E. G. Sochilin, Zhur. Organ. Khim., 14, 1323 (1978).

Variations of the above Scheme 1 are also readily prepared by the methods of Scheme 1' discussed above. These are shown here in Scheme 1'' as follows.

Scheme 1"

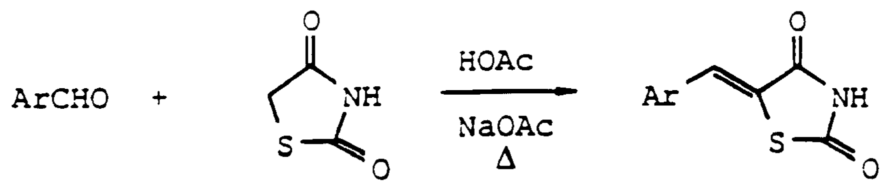


An alternate procedure for the preparation of the above noted oxazole I wherein X is 0 and Y is 0 is by an alkylation/hydrolysis procedure (Scheme 2') as follows:

**Scheme 2'**

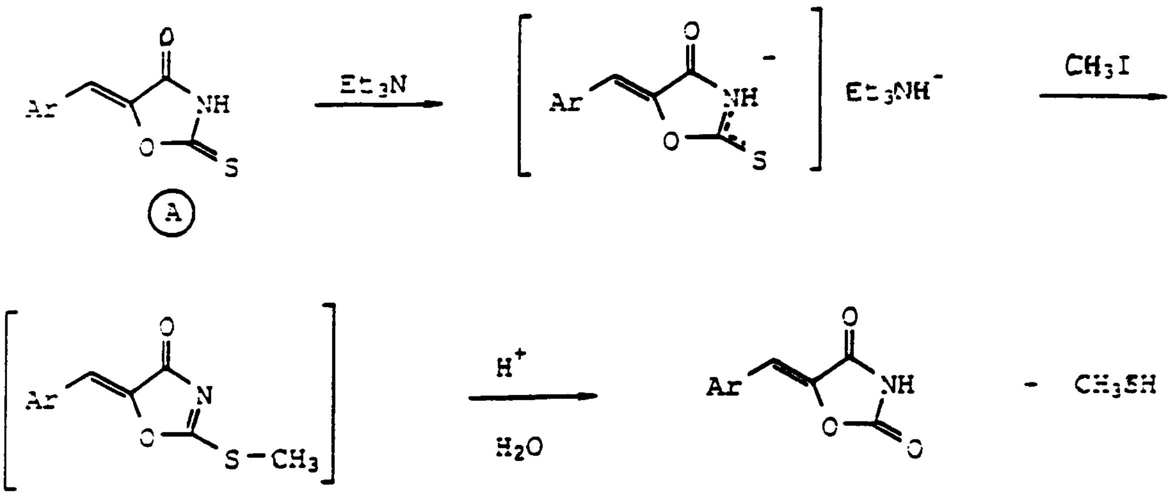


The procedure of Scheme 2' is essentially that of N. A. Shenberg, L. S. Guseva, A. I. Ginak, and E. G. Sochilin, Zhur. Organ. Khim., 14, 1323 (1978); and J. S. H. Davies, W. Hook, and F. Long, J. Chem. Soc., 30 (1950).

The compounds of formula I are useful both in the fee acid form, in the form of base salts where possible, and in the form of acid addition salts. The three forms are within the scope of the invention. In practice, use of the salt form amounts to use of the base form. Appropriate pharmaceutically acceptable salts within the scope of the invention are those derived from mineral acids such as hydrochloric acid and sulfuric acid; and organic acids such as methanesulfonic acid, benzenesulfonic acid, p-toluenesulfonic acid, and the like, giving the hydrochloride, sulfamate, methanesulfonate, benzenesulfonate, p-toluenesulfonate, and the like, respectively or those derived from bases such as suitable organic and inorganic bases. Examples of suitable inorganic bases for the formation of salts of compounds of this invention include the hydroxides, carbonates, and bicarbonates of ammonia, sodium, lithium, potassium, calcium, magnesium, aluminum, zinc, and the like.

Salts may also be formed with suitable organic bases. Bases suitable for the formation of pharmaceutically acceptable base addition salts with compounds of the present invention include organic bases which are nontoxic and strong enough to form such salts. These organic bases form a class whose limits are readily understood by those skilled in the art. Merely for purposes of illustration, the class may be said to include mono-, di-, and trialkylamines, such as methylamine, dimethylamine, and triethylamine; mono-, di-, or trihydroxyalkylamines such as mono-, di-, and triethanolamine; amino acids such as arginine, and lysine; guanidine; N-methylglucosamine; N-methylpiperazine; morpholine; ethylenediamine; N-benzylphenethylamine; tris(hydroxymethyl) aminomethane; and the like. (See for example, "Pharmaceutical Salts," J. Pharm. Sci., 66(1), 1-19 (1977).)

The acid addition salts of said basic compounds are prepared either by dissolving the free base of compound I in aqueous or aqueous alcohol solution or other suitable solvents containing the appropriate

acid or base and isolating the salt by evaporating the solution, or by reacting the free base of compound I with an acid as well as reacting compound I having an acid group thereon with a base such that the reactions are in an organic solvent, in which case the salt separates directly or can be obtained by concentration of the solution.

The base salts of compounds of formula I described above are prepared by reacting the appropriate base with a stoichiometric equivalent of the acid compounds of formula I to obtain pharmacologically acceptable base salts thereof.

The acid solution salts of said basic compounds are prepared either by dissolving the free base in aqueous or aqueous alcohol solution or other suitable solvents containing the appropriate acid and isolating the salt by evaporating the solution, or by reacting the free base and acid in an organic solvent, in which case the salt separates directly or can be obtained by concentration of the solution.

The compounds of this invention may also exist in hydrated or solvated forms.

The products of the reactions described herein are isolated by conventional means such as extraction, distillation, chromatography and the like.

The antiallergic and antiinflammatory activity of the compounds having the formula I of the present invention is determined by an assay showing inhibition of the release of histamine from human basophils (HHB). A description of the protocol of the HHB assay is found hereinafter.

Thus, pharmaceutical compositions are prepared from the compounds of formula I and salts thereof described as the present invention in unit dosage form comprising the compound in admixture with a pharmaceutically acceptable carrier appropriately selected from those known.

A physician or veterinarian of ordinary skill readily determines a subject who is exhibiting allergic or inflammatory symptoms. Regardless of the route of administration selected, the compounds of the present invention are formulated into pharmaceutically acceptable dosage forms by conventional methods known to the pharmaceutical art.

The compounds can be administered in such oral unit dosage forms such as tablets, capsules, pills, powders, or granules. They also may be administered rectally or vaginally in such forms as suppositories or bougies; they may also be introduced parenterally (e.g., subcutaneously, intravenously, or intramuscularly), using forms known to the pharmaceutical art. They are also introduced directly to an affected area (e.g., in the form of eye drops or by inhalation). For the treatment of allergic or inflammatory conditions such as erythema, the compounds of the present invention may also be administered topically in the form of ointments, creams, gels, or the like. In general, the preferred route of administration is orally.

An effective but nontoxic quantity of the compound I is employed in treatment. The ordinarily skilled physician or veterinarian will readily determine and prescribe the effective amount of the antiallergic or antiinflammatory agent to prevent or arrest the progress of the condition. The dosage regimen is selected in accordance with a variety of factors including the type, age, weight, sex, and medical condition of the mammal, the severity of symptoms of the disease being treated, the route of administration and particular compound of formula I employed. An ordinarily skilled physician or veterinarian will readily determine and prescribe the effective amount of the compound I to prevent or arrest the progress of the condition. In so proceeding, the physician or veterinarian could employ relatively low dosages at first, subsequently increasing the dose until a maximum response is obtained. For convenience, the total daily dosage may be divided and administered in portions during the day if desired. In so proceeding, the physician or veterinarian could employ relatively low dosages at first, subsequently increasing the dose until a maximum response is obtained.

Initial dosages of the compounds of the invention having formula I are ordinarily in the area of 10 mg up to 2 g per day orally, preferably 20 mg to 500 mg per dose orally, given from one to four times daily or as needed. When other forms of administration are employed, equivalent doses are administered.

A suitable dose of a compound of formula (I) or pharmacologically acceptable salt thereof for a mammal suffering from, or likely to suffer from any condition as described hereinbefore is 0.1 $\mu$g - 500 mg of the compound per kilogram body weight. In the case of systemic administration, the dose may be in the range of 0.5 to 500 mg of the compound per kilogram body weight, the most preferred dosage being 0.5 to 50 mg/kg of mammal body weight administered two or three times daily. In the case of topical administration, e.g., to the skin or eye, a suitable dose may be in the range 0.1 ng-100 ug of the compound per kilogram, typically about 0.1 $\mu$g/kg.

In the case of oral dosing for the treatment or prophylaxis of arthritis or inflammation in general, due to any course, a suitable dose of a compound of formula (I) or physiologically acceptable salt thereof, may be as specified in the preceding paragraph, but most preferably is from 1 mg to 10 mg of the compound per kilogram, the most preferred dosage being from 1 mg to 5 mg/kg of mammal body weight, for example, from 1 to 2 mg/kg.

It is understood that the compositions and methods of treatment of the present invention as described above also include the free acid, the pharmacologically acceptable base salts and acid addition salts of the compounds of formula I.

The following Examples further illustrate the invention, but are not meant to be limiting thereto.

EXAMPLE 1

5-[(4-Hydroxy-3,5-dimethoxyphenyl)methylene]-2,4-thiazolidinedione

A mixture of 3,5 dimethoxybenzaldehyde (4,8 g, 26 mmoles) 2,4-thiazolidinedione (2,9 g, 25 mmoles) sodium acetate (8.4 g, 102 mmoles), and acetic acid (50 ml) is stirred under an inert atmosphere and heated to reflux. After 4 hours the mixture is stirred into water (250 ml) and the precipitate is filtered off, rinsed successively with water (3X), ethanol (2X), and ether (2X), and dried to afford the pure product (1,7 g), mp 248-249 °C.

EXAMPLE 2

5-[[3,5-Bis(1,1-dimethylethyl)-4-hydroxyphenyl]methylene]-2,4-thiazolidinedione

A mixture of 3,5-di-t-butyl-4-hydroxybenzaldehyde (6.5 g, 27 mmoles), 2,4-thiazolidinedione (3.0 g, 26 mmoles), sodium acetate (7.6 g, 93 mmoles), and acetic acid (40 ml) is stirred under an inert atmosphere and heated to reflux. After 78 hours the mixture is stirred into water (300 ml), and the precipitate is filtered off, rinsed three times with water, dried, and recrystallized from ethanol. The product is stirred in 1 N NaOH (50 ml) and extracted several times with dichloromethane. The aqueous solution is acidified with acetic acid, stirred for 1-2 hours, and the precipitate is filtered off, rinsed three times with water and dried to afford the pure product (2.3 g), mp 238-240 °C.

The following compound is prepared by the method described in Example 2.

Example 3: 5-[4-hydroxy-3-methoxyphenyl)-methylene]-2,4-thiazolidinedione, mp. 225-227 °C.

INTERMEDIATE

2-Thioxo-4-oxazolidinone

A solution of 72.9 g (0.75 mole) of potassium thiocyanate and 48.9 g (0.75 mole) of potassium cyanide in 85 ml of water was stirred in an ice bath and treated over 30 minutes with 60 ml (~0.80 mole) of 37% aqueous formaldehyde solution. The rate of addition was adjusted to maintain a reaction temperature of ≦10°. The mixture was stirred for an additional 90 minutes, then treated over 1 hour with 156 ml of concentrated hydrochloric acid (reaction temperature maintained at ≦10°). The mixture was stirred for 16 hours as it slowly warmed to room temperature. The inorganic solids were filtered and discarded, and the filtrate was warmed on the steam bath for 90 minutes. The reaction mixture was again filtered, and the filtrate was extracted with ether (5X 200 ml). The combined extracts were dried (anhydrous sodium sulfate) and evaporated to an oil, which slowly crystallized. The solid was washed with hexane to yield 22.7 g (26% yield), mp 106-109°. A sample recrystallized from ethyl acetate/hexane was analytically pure, mp 110-112° (a mp of 113° is given by N. K. Ushenko and T. E. Gorizdra, Ukrain. Khim. Zhur., 16, 545 (1950)).

EXAMPLE 4

5-[(4-Hydroxy-3-methoxyphenyl)methylene]-2-thioxo-4-oxazolidinone

A mixture of 3.25 g (0.021 mole) of 4-hydroxy-3-methoxybenzaldehyde, 2.34 g (0.020 mole) of 2-thioxo-4-oxazolidinone, 5.8 g (0.071 mole) of sodium acetate and 15 ml of acetic acid was stirred and heated at reflux under a nitrogen atmosphere for 2 hours. The mixture was then stirred at room temperature for 16 hours, and added to 300 g of ice/water. The precipitated solid was filtered, washed with water, and recrystallized from aqueous methanol/N,N-dimethylformamide to yield 2.4 g (49% yield) of the oxazole product. A sample recrystallized a second time as above was analytically pure, mp 240°-dec. (A mp of 237-238° is given by T. E. Gorizdra and S. N. Baranov, Zhur. Obshchei Khim., 26, 3092 (1956)).

| Calcd. for $C_{11}H_9NO_4S \cdot 0.25H_2O$: | | | | |
|---|---|---|---|---|
| | C, 51.65; | H, 3.74; | N, 5.48; | S, 12.54 |
| Found: | C, 51.54; | H, 3.97; | N, 5.38; | S, 12.17 |

Also prepared by the procedure described in Example 4 utilizing the appropriate aldehyde intermediate were:

EXAMPLE 5

5-[(3,4-Dimethoxyphenyl)methylene]-2-thioxo-4-oxazolidinone

mp 249°-dec.

| Calcd. for $C_{12}H_{11}NO_4S$: | | | | |
|---|---|---|---|---|
| | C, 54.32; | H, 4.18; | N, 5.28; | S, 12.09 |
| Found: | C, 53.92; | H, 4.10; | N, 5.22; | S, 11.84. |

EXAMPLE 6

5-[(4-Hydroxy-3,5-dimethoxyphenyl)methylene]-2-thioxo-4-oxazolidinone

mp 250°-dec. (recrystallized from aqueous acetonitrile/N,N-dimethylformamide).

| Calcd. for $C_{12}H_{11}NO_5S$: | | | | |
|---|---|---|---|---|
| | C, 51.24; | H, 3.94; | N, 4.98; | S, 11.40 |
| Found: | C, 51.00; | H, 3.78; | N, 5.30; | S, 11.45. |

EXAMPLE 7

5-[(4-Hydroxy-3,5-dimethoxyphenyl)methylene]-2-thioxo-4-oxazolidinone

mp 253°-dec.

| Calcd. for $C_{12}H_{11}NO_3S$: | | | | |
|---|---|---|---|---|
| | C, 57.81; | H, 4.45; | N, 5.62; | S, 12.86 |
| Found: | C, 57.84; | H, 4.49; | N, 5.56; | S, 12.54. |

EXAMPLE 8

5-[(3-Bromo-4-hydroxy-5-methoxyphenyl)methylene]-2-thioxo-4-oxazolidinone

mp 260-262°

| Calcd. for $C_{11}H_8BrNO_4S$: | | | | | |
|---|---|---|---|---|---|
| | C, 40.01; | H, 2.44; | N, 4.24; | S, 9.71; | Br, 24.20 |
| Found: | C, 40.18; | H, 2.34; | N, 4.07; | S, 9.61; | Br, 24.02. |

EXAMPLE 9

2-Thioxo-5-[(2,4,5-trimethoxyphenyl)methylene]-4-oxazolidinone

mp 265°-dec. (recrystallized from aqueous methanol/N,N-dimethylformamide).

| Calcd. for $C_{13}H_{13}NO_5S$: | | | | |
|---|---|---|---|---|
| | C, 52.87; | H, 4.44; | N, 4.74; | S, 10.86 |
| Found: | C, 52.55; | H, 4.30; | N, 4.79; | S, 10.47. |

EXAMPLE 10

2-Thioxo-5-[(3,4,5-trimethoxyphenyl)methylene]-4-oxazolidinone

mp 230°-dec. (recrystallized from aqueous methanol/N,N-dimethylformamide).

| Calcd. for $C_{13}H_{13}NO_5S$: | | | | |
|---|---|---|---|---|
| | C, 52.87; | H, 4.44; | N, 4.74; | S, 10.86 |
| Found: | C, 52.81; | H, 4.32; | N, 4.62; | S, 10.39. |

EXAMPLE 11

5-[(4-Hydroxy-3,5-dimethoxyphenyl)methylene]-2-thioxo-4-oxazolidinone, disodium salt

A suspension of 3.1 g (0.011 mole) of 5-[(4-hydroxy-3,5-dimethoxyphenyl)methylene]-2-thioxo-4-oxazolidinone in 20 ml of water was treated with a solution of 11.0 ml of 2.00 N aqueous sodium hydroxide solution. The mixture was warmed briefly on the steam bath until nearly homogenous, then filtered warm. The cooled filtrate was subjected to vacuum freeze-drying to yield 3.5 g (98% yield) of the analytically pure salt product, mp >265°.

| Calcd. for $C_{12}H_9NO_5SNa_2 \cdot 0.6\ H_2O$: | | | |
|---|---|---|---|
| | C, 42.89; | H, 3.06; | N, 4.17 |
| Found: | C, 42.67; | H, 2.88; | N, 4.00. |

Also prepared by the above procedures was:

EXAMPLE 12

5-[(4-Hydroxy-3-methoxyphenyl)methylene]-2-thioxo-4-oxazolidinone, disodium salt

mp >270°

| Calcd. for $C_{11}H_7NO_4SNa_2 \cdot 1\ H_2O$: | | | |
|---|---|---|---|
| | C, 42.18; | H, 2.90; | N, 4.47 |
| Found: | C, 41.84; | H, 2.52; | N, 4.37. |

The following compounds are prepared according to the procedure described in Example 1 using appropriate corresponding starting materials.

EXAMPLE 13

5-[(3-Bromo-4-hydroxyphenyl)methylene]-2-thioxo-4-oxazolidinone, mp 240° (dec). Prepared according to the procedure described in Example 7.

EXAMPLE 14

5-[[3,5-Bis(1,1-dimethylethyl-4-hydroxyphenyl]methylene-2-thioxo-4-oxazolidinone

A mixture of 14.1 g (0.060 mole) of 3,5-di-tert-butyl-4-hydroxybenzaldehyde, 7.0 g (0.060 mole) of 2-thioxo-4-oxazolidinone, 17.4 g (0.21 mole) of sodium acetate, and 75 ml of acetic acid is stirred and heated at reflux under a nitrogen atmosphere for 20 hours. The cooled reaction mixture is added to 900 g of ice/water and the precipitated product filtered and washed with water. There is obtained 16.2 g (81% yield) of the oxazole product, suitable for further reaction.

A sample of the above crude product is chromatographed over silica gel, using elution with 2.5% ethyl acetate in dichloromethane followed by 25% ethyl acetate. The chromatography product is recrystallized from aqueous acetonitrile to yield the analytically pure oxazole, mp 240°C dec.

| Calcd. for $C_{18}H_{23}NO_3S$: | | | |
|---|---|---|---|
| | C, 64.83; | H, 6.95; | N, 4.20 |
| Found: | C, 65.00; | H, 6.95; | N, 4.17. |

EXAMPLE 15

5-[[3,5-Bis(1,1-dimethylethyl-4-hydroxyphenyl]methylene]-2,4-oxazolidinedione

A solution of 10.0 g (0.030 mole) of 5-[[3,5-bis(1,1-dimethylethyl-4-hydroxyphenyl]methylene-2-thioxo-4-oxazolidinone in 150 ml of tetrahydrofuran is cooled in ice and treated with 4.2 ml (3.0 g, 0.030 mole) of triethylamine.

The mixture is stirred with ice cooling for one hour, then treated with 10.0 ml (22.8 g, 0.16 mole) of iodomethane. The ice bath is removed and the mixture stirred for an additional 24 hours. The reaction mixture is filtered and the filter cake is washed several times with fresh tetrahydrofuran. The combined filtrates are evaporated and the residue is chromatographed (silica gel, 2% methanol in dichloromethane elution) to yield 4.4 g (42% yield) of the purified 2-methylthio- intermediate. This material is hydrolyzed without further purification.

A solution of 3.0 g (0.086 mole) of the above 2-methylthio-intermediate in 90 ml of ethanol is diluted with 30 ml of water and treated dropwise over 10 minutes with 5.0 ml of concentrated hydrochloric acid. The reaction mixture is stirred for 24 hours and the precipitated product is filtered and washed with hexane. The crude yield is 1.44 g (53%). A sample recrystallized from ethyl acetate/hexane yielded the analytically pure oxazolidinedione, mp 239°C dec.

| Calcd. for $C_{18}H_{23}NO_4$: | | | |
|---|---|---|---|
| | C, 68.12; | H, 7.31; | N, 4.41 |
| Found: | C, 68.22; | H, 7.29; | N, 4.05. |

Also prepared by the above procedure, utilizing the appropriate 2-thioxo-4-oxazolidinone intermediate are:

EXAMPLE 16

5-[(4-Hydroxy-3,5-dimethoxyphenyl)methylene]-2,4-oxazolidinedione

mp 268° dec. (recrystallized from acetonitrile/DMF/water).

| Calcd. for $C_{12}H_{11}NO_6$: | | | |
|---|---|---|---|
| | C, 54.34; | H, 4.18; | N, 5.28 |
| Found: | C, 54.00; | H, 4.17; | N, 5.15. |

EXAMPLE 17

5-[(4-Hydroxy-3,5-dimethylphenyl)methylene]-2,4-oxazolidinedione

mp 275°C dec. (recrystallized from acetonitrile/DMF/water).

| Calcd. for $C_{12}H_{11}NO_4 \cdot 0.5H_2O$: | | | |
|---|---|---|---|
| | C, 59.50; | H, 4.99; | N, 5.78 |
| Found: | C, 59.31; | H, 5.02; | N, 5.72. |

The usefulness of the compounds of the present invention as inhibitors of histamine release is demonstrated by the following assay. The assay is essentially as generally accepted among the ordinarily skilled artisans to show activity having usefulness to treat the diseases or conditions as set out in the present invention. A description of the procedure follows.

HISTAMINE RELEASE FROM HUMAN BASOPHILS (hereinafter HHB)

The HHB assay quantitates active histamine release, and its inhibition by drugs, from basophils of human blood. Thus, the assay provides evaluation of the compounds of formula I for treating the conditions or diseases as is the present invention. As described herein the assay includes modifications of the method described by R. P. Siraganian in "An Automated Continuous-Flow System for the Extraction and Fluorometric Analysis of Histamine", Anal. Biochem., 57, 383-394 (1974).

EXAMPLE 18

5-[[3,5-Bis(1,1-dimethylethyl)-4-hydroxyphenyl]methylene]-2-thioxo-4-oxazolidinone, choline salt

A solution of 2.45 g (0.0069 mole) of 46.6% aqueous choline bicarbonate in 50 ml of methanol is stirred and treated over 5 minutes with 2.31 g (0.0069 mole) of 5[[3,5-bis(1,1-dimethylethyl)-4-hydroxyphenyl]-methylene]-2-thioxo-4-oxazolidinone. The reaction mixture is warmed for a few minutes to reflux on the steam bath, and filtered hot. The cooled filtrate is evaporated, and the residue recrystallized from acetone/tert-butyl methyl ether to yield 2.1 g (70% yield) of the analytically pure choline salt, mp 167° dec.

| Calc. for $C_{23}H_{36}N_2O_4S$: | | | |
|---|---|---|---|
| | C, 63.27; | H, 8.31; | N, 6,42 |
| Found: | C, 63.22; | H, 8.21; | N, 6.28 |

EXAMPLE 19

5-[[3,5-bis(1,1-dimethylethyl)-4-hydroxyphenyl]methylene]-2,4-thiazolidinedione, choline salt

A solution of 46.6% aqueous choline bicarbonate (10.55 g, 29.8 mmoles) is added dropwise under nitrogen to a stirred suspension of 5-[[3,5-bis(1,1-dimethylethyl)-4-hydroxyphenyl]-methylene]-2,4-thiazolidinedione (10.0 g, 30.0 mmoles) in ethanol (100 mls). The solution is gradually warmed to reflux for one hour, and is then cooled and stripped of solvent by rotary evaporator. Two 50 ml portions of ethanol are successively mixed with and stripped from the residue which is then thoroughly dried under vacuum. The residue is triturated in diethyl ether, filtered off, washed twice with ether, and dried under vacuum at 80 °C overnight to afford the crystalline choline salt (12.8 g), mp 215 °C dec.

| Calc. for $C_{18}H_{22}NO_3S \cdot C_5H_{14}NO$: | | | |
|---|---|---|---|
| | C, 63.27; | H, 8.31; | N, 6,42 |
| Found: | C, 63.12; | H, 8.28; | N, 6.17 |

EXAMPLE 20

5-[(3-Methoxyphenyl)methylene]-2-thioxo-4-oxazolidinone

mp 215 °-dec (recrystallized from methanol (N,N-dimethylformamide).

| Calcd. for $C_{11}H_9NO_3S$: | | | | |
|---|---|---|---|---|
| | C, 56.15; | H, 3.86; | N, 5.95; | S, 13.63 |
| Found: | C, 56.34; | H, 3.87; | N, 5.95; | S, 13.51. |

EXAMPLE 21

5-[(4-Methoxyphenyl)methylene]-2-thioxo-4-oxazolidinone

mp 205-207 ° (recrystallized from aqueous methanol/N,N-dimethylformamide). (A mp of 192 ° is given by N. K. Ushenko and T. E. Gorizdra, Ukrain Khim. Zhur., 16, 545 (1950)).

| Calcd. for $C_{11}H_9NO_3S$: | | | | |
|---|---|---|---|---|
| | C, 56.15; | H, 3.86; | N, 5.95; | S, 13.63 |
| Found: | C, 56.10; | H, 3.88; | N, 6.03; | S, 13.62. |

METHODS

Preparation of Leukocytes

Blood is drawn from allergic donors (chosen on the basis of adequate histamine release induced by a challenge), using standard venipuncture methods, into Vacutainers with EDTA in water as anticoagulant. The blood samples are placed briefly on a rotary mixer. The blood is mixed with Hespan (hydroxy ethyl starch, 0.5 ml per 1.0 ml of blood), the tube inverted several times to mix and then left undisturbed at room temperature until a sharp separation is observed between the settled red cells and the leukocyte and platelet-rich plasma. This usually occurs within 35-45 minutes.

The plasma fraction is removed and centrifuged for 12 minutes at 4 °C at 1050 RPM (100 Xg). The platelets remain in the plasma and are discarded. The pelleted leukocytes are shaken gently to disrupt the cell button and washed twice with HA buffer containing 0.005 M EDTA and resuspended in HACM buffer to approximately one-quarter the original blood volume. A sample is prepared for Hematology, where a total

white blood cell and platelet count is done using a Coulter Counter.

Protocol Design

Aliquots (0.1 ml) of cells are added to triplicate assay tubes containing 0.4 ml of either 6% perchloric acid (for total histamine content), vehicle control (for spontaneous release), or drug. The tubes are incubated at room temperature for 8 minutes, and then placed in a 37°C water bath for 2 more minutes. Buffer or challenge agents (at 37°C) are added to the tubes and they are incubated for an additional 45 minutes at 37°C in a shaking water bath. The tubes are then spun at 2000 RPM (1200 g) for 3 minutes to pellet the cells and the supernatants are removed and assayed for histamine by the fluorometric method.

Drug Preparation

A 300 µM stock solution of each test compound is prepared in distilled water, using 0.5 ml DMSO/100 ml and/or 0.2 ml of 1N NaOH or HCl and/or heat to aid in dissolution. Five ml of the stock solution is diluted (1:2) with 5 ml of two times concentrated HACM buffer to yield the stock working concentration of 150 µM. When added to the cells and stimulus, a final test concentration of 100 µM drug results. Further dilutions are made with HACM buffer for 33, 10, 3.3, 1.0 µM, etc.

Challenge Agent Preparation

Short ragweed and house dust extracts (Greer Laboratories, Inc.) are supplied as aqueous extracts in stock concentrations of 40,000 and 10,000 protein nitrogen units per milliliter (PNU/ml), respectively. Aqueous solutions of anti-IgE antisera (rabbit-raised antibody) are purchased from Dako via Accurate Chemicals. The aqueous solutions of ragweed, house dust, and anti-IgE are diluted 1:2 with two times concentrated HACM and then further diluted with HACM to yield final stock concentrations of 6000 PNU/ml for ragweed and house dust and 1:50 dilution for the anti-IgE antisera. Further dilutions for working solutions are made in HACM buffer. All stock and working solutions are stored at 4°C. Working solutions comprise 1/6 of the final volume in the cell reaction, therefore, working solutions of challenge agents are made up six times the required final concentration.

In each experiment, cells are challenged according to the previously determined sensitivity of that donor to the particular challenge agent. Short ragweed and house dust concentrations are expressed in PNU/ml, and anti-IgE antisera is expressed as dilutions, e.g., IE-5 (1:100,000), 3E-5 (1:30,000), and IE-4 (1:10,000).

Calculation and Interpretation of Results

The total histamine concentration in the "total" (acid-treated) samples must be 15 ng/ml to be acceptable. Spontaneous release of histamine from the cells should not exceed 15% of the total histamine, and is frequently < 5%. The maximum percentage histamine released varies with the donor. The net amount released by the challenge agent must exceed 25% of the total cellular histamine to confidently assess inhibition by test compounds. Spontaneous histamine release is subtracted from both "totals" and challenged cells to calculate net percent release. Percent inhibition is shown in the Table and is calculated using the following formula:

$$1-\left[\frac{\text{Mean net \% release treated samples}}{\text{Mean net \% release for challenged control}}\right] \times 100 = \text{\% inhibition}$$

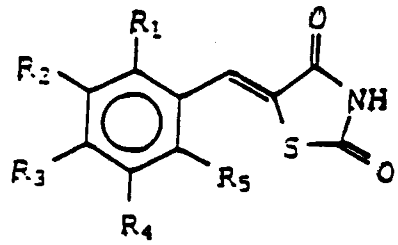

| Ex | $R_1$ | $R_2$ | $R_3$ | $R_4$ | $R_5$ | HHB % Inhibition of Histamine Release at 33 $\mu$M |
|---|---|---|---|---|---|---|
| 1 | H | MeO | OH | MeO | H | 60 |
| 2 | H | tBu | OH | tBu | H | 49 |
| 3 | H | OMe | OH | H | H | 81 |

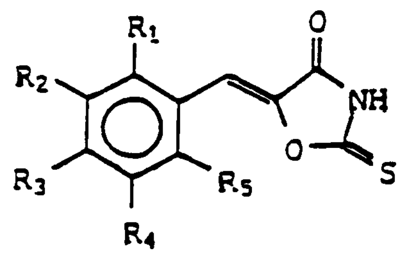

| Ex | $R_1$ | $R_2$ | $R_3$ | $R_4$ | $R_5$ | HHB % Inhibition of Histamine Release at 33 $\mu$M |
|---|---|---|---|---|---|---|
| 4 | H | MeO | OH | H | H | 77 |
| 5 | H | MeO | MeO | H | H | 43 |
| 6 | H | MeO | OH | Meo | H | 89 |
| 7 | H | Me | OH | Me | H | 88 |
| 8 | H | Br | OH | MeO | H | * |
| 9 | MeO | H | MeO | MeO | H | * |
| 10 | H | MeO | MeO | MeO | H | 76 |
| 13 | H | Br | OH | H | H | 66 |
| * Indicates not active at 33 $\mu$M. | | | | | | |

E Enhanced release at this concentration.
MeO is methoxy.
$OCHMe_2$ is isopropoxy.
$OCH_2Ph$ is benzyloxy.
tBu is tertiarybutyl.
$Me_2NCH_2$ is dimethylamine.

Activity is measured as % inhibition of histamine release from human basophils challenged with anti-IgE at a 33 uM concentration of drug.

The $IC_{50}$ is calculated to give the $\mu$M necessary to provide 50% inhibition.

The usefulness of the compounds of the present invention as inhibitors of the 5-lipoxygenase enzyme, cyclooxygenase, or in treating related diseases or conditions may be demonstrated by their effectiveness in various standard test procedures. A description of each procedure follows.

ARBL/ARBC Whole Cell 5-Lipoxygenase and Cyclooxygenase Assays

Materials

The rat basophilic Leukemia cell line (RBL-1) was obtained from the American Type Culture Collection (Rockville, MD).

Radioimmunoassay (RIA) kits of $LTB_4$ and $PGF_{2\alpha}$ were obtained from Amersham (Arlington Heights, IL) and Seragen (Boston, MA), respectively.

All tissue culture media were obtained from GIBCO (Grand Island, NY).

Method

RBL-1 cells are grown in suspension culture in Eagle's minimum essential medium supplemented with 12% fetal bovine serum at 37°C in an incubator supplied with air-5% carbon dioxide. Cells are harvested by centrifugation. They are washed with cold phosphate buffered saline pH 7.4 (PBS; NaCl, 7.1 g; $Na_2HPO_4$, 1.15 g; $KH_2PO_4$, 0.2 g; and KCl, 0.2 g/l). Cells are finally suspended in PBS containing 1.0 mM calcium at a density of 2 x $10^6$ cells/ml. Cells are incubated with and without test agent (in DMSO) (1% DMSO is without effect on arachidonic acid metabolism) for ten minutes at room temperature. Calcium ionophore A23187 (5 $\mu$M) is added and cells are incubated for seven minutes at 37°C. The reaction is stopped by chilling the tubes on ice for ten minutes. Cells are separated by centrifugation and the supernatant is stored at -20°. Aliquots (100 $\mu$l) are analyzed for $LTB_4$ and $PGF_{2\alpha}$ using radioimmunoassay kits as provided by the supplier.

Table 1 contains biochemical data obtained from this whole cell assay as $IC_{50}$s which are calculated as the amount of test compound causing 50% inhibition of $LTB_4$ or $PGF_{2\alpha}$ formation.

Carrageenan-Induced Rat Foot Paw Edema-2 (CEF-2) Assay: Protocol

Carrageenan solution (1% w/v) is prepared by dissolving 100 mg carrageenan (Marine Colloidal Div., Springfield, NJ) in 10 ml of sterile saline (0.9%) solution (Travenol). The solution is vortexed for 30 to 45 minutes. Animals are dosed with compound one hour before carrageenan challenge. Foot paw edema is induced by injecting 0.10 ml of the 1% carrageenan subcutaneously into the plantar portion of the right hind paw of each rat under light anesthesia. Initial foot paw volume is measured immediately following carrageenan challenge using mercury plethysmography (Buxco Electronics). Edemia is measured five hours after carrageenan. The difference between the five-hour and the initial paw volume is expressed as delta edema. The delta edema for each test group of animals is used to calculate the percent inhibition of edema achieved by the compound at the test dose compared with the vehicle control group. The $ID_{40}$ (the dose at which swelling is inhibited by 40%) is calculated by probit analysis for the dose at which 40 percent inhibition occurs.

TABLE II

| Example No. | ARBL[(1)] | ARBC[(2)] | CFE[(3)] |
|---|---|---|---|
| 2 | 100 | 95 | 31.8 (1)<br>39.8 (3)<br>53.7 (10)<br>58.8 (30) |
| 14 | 92 | 86 | 44.1 (1)<br>41.1 (3)<br>32.1 (10)<br>40.4 (30) |
| 15 | 95 | 89 | 18.5 (1)<br>23.5 (3)<br>36.8 (10)<br>39.4 (30) |
| 16 | 67 | N[(4)] | |

(1) Percent inhibition of cellular 5-lipoxygenase at 16 $\mu$M.
(2) Percent inhibition of cellular 5-cyclooxygenase at 16 $\mu$M.
(3) Percent inhibition of carrageenan footpad edema (CFE) test at various doses (mg/kg) of test drug.
(4) N inactive at screening concentration.

Accordingly, the present invention also includes a pharmaceutical composition for treating one of the above diseases or conditions comprising an antidisease or anticondition effective amount of a compound of the formula I as defined above together with a pharmaceutically acceptable carrier.

The present invention further includes a method for treating one of the above named diseases or conditions in mammals, including man, suffering therefrom comprising administering to such mammals either orally or parenterally, preferably oral, a corresponding pharmaceutical composition containing a compound of formula I as defined above in appropriate unit dosage form.

For preparing pharmaceutical compositions from the compounds described by this invention, inert, pharmaceutically acceptable carriers can be either solid or liquid. Solid form preparations include powders, tablets, dispersible granules, capsules, cachets, and suppositories. A solid carrier can be one or more substances which may also act as diluents, flavoring agents, solubilizers, lubricants, suspending agents, binders or tablet disintegrating agents; it can also be encapsulating material. In powders, the carrier is a finely divided active compound. In the tablet the active compound is mixed with carrier having the necessary binding properties in suitable proportions and compacted in the shape and size desired. The powders and tablets preferably contain from 5 or 10 to about 70 percent of the active ingredient. Suitable solid carriers are magnesium carbonate, magnesium stearate, talc, sugar, lactose, pectin, dextrin, starch, gelatin, tragacanth, methylcellulose, sodium carboxymethylcellulose, a low melting wax, cocoa butter, and the like. The term "preparation" is intended to include the formulation of the active compound with encapsulating material as carrier providing a capsule in which the active component (with or without other carriers) is surounded by carrier, which is thus in association with it. Similarly, cachets are included. Tablets, powders, cachets, and capsules can be used as solid dosage forms suitable for oral administration.

For preparing suppositories, a low melting wax such as a mixture of fatty acid glycerides or cocoa butter is first melted, and the active ingredient is dispersed homogeneously therein as by stirring. The molten homogeneous mixture is then poured into convenient sized molds, allowed to cool and thereby to solidify.

Liquid form preparations include solutions, suspensions, and emulsions. As an example may be mentioned water or water-propylene glycol solutions for parenteral injection. Liquid preparations can also be formulated in solution in aqueous polyethylene glycol solution. Aqueous solutions suitable for oral use can be prepared by dissolving the active component in water and adding suitable colorants, flavors, stabilizing and thickening agents as desired. Aqueous suspensions suitable for oral use can be made by dispersing the finely divided active component in water with viscous material, i.e., natural or synthetic gums, resins, methylcellulose, sodium carboxymethylcellulose, and other well-known suspending agents.

Also included are solid form preparations which are intended to be converted, shortly before use, to liquid form preparations for either oral or parenteral administration. Such liquid forms include solutions, suspensions, and emulsions. These particular solid form preparations are most conveniently provided in unit dose form and as such are used to provide a single liquid dosage unit. Alternately, sufficient solid may be provided so that after conversion to liquid form, multiple individual liquid doses may be obtained by measuring predetermined volumes of the liquid form preparation as with a syringe, teaspoon, or other volumetric container. When multiple liquid doses are so prepared, it is preferred to maintain the unused portion of said liquid doses at low temperature (i.e., under refrigeration) in order to retard possible decomposition. The solid form preparations intended to be converted to liquid form may contain, in addition to the active material, flavorants, colorants, stabilizers, buffers, artificial and natural sweeteners, dispersants, thickeners, solubilizing agents, and the like. The liquid utilized for preparing the liquid form preparation may be water, isotonic water, ethanol, glycerine, propylene glycol, and the like as well as mixtures thereof. Naturally, the liquid utilized will be chosen with regard to the route of administration, for example, liquid preparations containing large amounts of ethanol are not suitable for parenteral use.

Preferably, the pharmaceutical preparation is in unit dosage form. In such form, the preparation is subdivided into unit doses containing appropriate quantities of the active component. The unit dosage form can be a packaged preparation, the package containing discrete quantities of preparation, for example, packeted tablets, capsules, and powders in vials or ampoules. The unit dosage form can also be a capsule, cachet, or tablet itself or it can be the appropriate number of any of these in packaged form.

The quantity of active compound in a unit dose of preparation may be varied or adjusted from 1 mg to 500 mg preferably to 1 to 50 mg according to the particular application and the potency of the active ingredient. The compositions can, if desired, also contain other compatible therapeutic agents.

In therapeutic use as described above, the dosages may be varied depending upon the requirements of the patient, the severity of the condition being treated, and the compound being employed. Determination of the proper dosage for a particular situation is within the skill of the art. Generally, treatment is initiated with the smaller dosages which are less than the optimum dose of the compound. Thereafter the dosage is increased by small increments until the optimum effect under the circumstances is reached. For convenience, the total daily dosage may be divided and administered in portions during the day if desired.

In addition to the compounds of formula I, the pharmaceutical compositions can also contain other active ingredients, such as cyclooxygenase inhibitors, nonsteroidal antiinflammatory drugs (NSAIDs), peripheral analgesic agents such as zomepirac, diflunisal, and the like. The weight ratio of the compound of the formula I to the second active ingredient may be varied and will depend upon the effective dose of each ingredient. Generally, an effective dose of each will be used. Thus, for example, when a compound of the formula I is combined with an NSAID, the weight ratio of the compound of the formula I to the NSAID will generally range from 1000:1 to 1:1000, preferably 200:1 to 1:200. Combinations of a compound of the formula I and other active ingredients will generally also be within the aforementioned range, but in each case, an effective dose of each active ingredient should be used.

Combinations of a compound of the formula I and other active ingredients will generally be in the aforementioned ratios.

NSAIDs can be characterized into five groups:

(1) the propionic acid derivatives;

(2) the acetic acid derivatives;

(3) the fenamic acid derivatives;

(4) the biphenylcarboxylic acid derivatives; and

(5) the oxicams

or a pharmaceutically acceptable salt thereof.

The propionic acid derivatives which may be used comprise: ibuprofen, ibuprufen aluminum, indoprofen, ketoprofen, naproxen, benoxaprofen, flurbiprofen, fenoprofen, fenbufen, pirprofen, carprofen, oxaprozin, pranoprofen, miroprofen, tioxaprofen, suprofen, alminoprofen, tiaprofen, fluprofen, and bucloxic acid. Structurally related propionic acid derivatives having similar analgesic and antiinflammatory properties are also intended to be included in this group.

Thus, "propionic acid derivatives" as defined herein are nonnarcotic analgesics/nonsteroidal antiinflammatory drugs having a free $-CH(CH_3)COOH$ or $-CH_2CH_2COOH$ group (which optionally can be in the form of a pharmaceutically acceptable salt group, e.g., $-CH(CH_3)COO^-Na^+$ or $-CH_2CH_2COO^-Na^+$), typically attached directly or via a carbonyl function to a ring system, preferably to an aromatic ring system.

The acetic acid derivatives which may be used comprise: indomethacin, which is a preferred NSAID, sulindac, tolmetin, zomepirac, diclofenac, fenclofenac, alclofenac, ibufenac, isoxepac, furofenac, tiopinac, zidometacin, acemetacin, fentiazac, clidanac, oxpinac, and fenclozic acid. Structurally related acetic acid

derivatives having similar analgesic and antiinflammatory properties are also intended to be encompassed by this group.

Thus, "fenamic acid derivatives" as defined herein are nonnarcotic analgesics/nonsteroidal antinflammatory drugs which contain the basic structure:

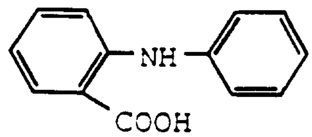

which can bear a variety of substituents and in which the free -COOH group can be in the form of a pharmaceutically acceptable salt group, e.g., $-COO^{-}Na^{+}$.

The biphenylcarboxylic acid derivatives which can be used comprise: diflunisal and flufenisal. Structurally related biphenylcarboxylic acid derivatives having similar analgesic and antiinflammatory properties are also intended to be encompassed by this group.

Thus, "biphenylcarboxylic acid derivatives" as defined herein are nonnarcotic analgesics/nonsteroidal antiinflammatory drugs which contain the basic structure:

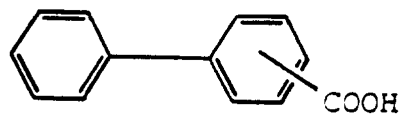

which can bear a variety of substituents and in which the free -COOH group can be in the form of a pharmaceutically acceptable salt group, e.g., $-COO-Na^{+}$.

The oxicams which can be used in the present invention comprise: piroxicam, sudoxicam, isoxicam, and 4-hydroxyl-1,2-benzothiazine 1,1-dioxide 4-(N-phenyl)-carboxamide. Structurally related oxicams having similar analgesic and antiinflammatory properties are also intended to be encompassed by this group.

Thus, "oxicams" as defined herein are nonnarcotic analgesica/nonsteroidal antiinflammatory drugs which have the general formula:

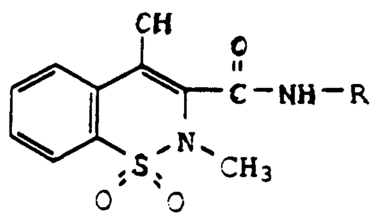

wherein R is an aryl or heteroaryl ring system.

The following NSAIDs may also be used: acemetacin, alminoprofen, amfenac sodium, aminoprofen, anitrazafen, antrafenine, auranofin, bendazac lysinate, benzydamine, beprozin, broperamole, bufezolac, carprofen, cinmetacin, ciproquazone, clidanac, cloximate, dazidamine, deboxamet, delmetacin, detomidine, dexindoprofen, diacerein, di-fisalamine, difenpyramide, emorfazone, enfenamic acid, enolicam, epirizole, etersalate, etodolac, etofenamate, fanetizole mesylate, fenclofenac, fenclorac, fendosal, fenflumizole, fentiazac, feprazone, floctafenine, flunixin, flunoxaprofen, fluproquazone, fopirtoline, fosfosal, furcloprofen, furofenac, glucametacin, guaimesal, ibuproxam, isofezolac, isonixim, isoprofen, isoxepac, isoxicam, lefetamine HCl, leflunomide, lofemizole, lonazolac calcium, lotifazole, loxoprofen, lysin, clonixinate, meclofenamate sodium, meseclazone, microprofen, nabumetone, nictindole, nimesulide, orpanoxin, oxametacin, oxapadol, oxaprozin, perisoxal citrate, pimeprofen, pimetacin, piproxen, pirazolac, pirfenidone, pirprofen, pranoprofen, proglumetacin maleate, proquazone, pyridoxiprofen, sudoxicam, suprofen, talmetacin, talniflumate, tenoxicam, thiazolinobutazone, thielavin B, tiaprofenic acidd, tiaramide HCl, tiflamizole,

timegadine, tioxaprofen, tolfenamic acid, tolpadol, tryptamid, ufenamate, and zidometacin.

Finally, NSAIDs which may also be used include the salicylates, specifically aspirin, and the phenylbutazones, and pharmaceutically acceptable salts thereof.

Pharmaceutical compositions comprising the formula I compounds may also contain as the second active ingredient, antihistaminic agents such as benadryl, dramamine, histadyl, phenergan, and the like. Alternatively, they may include prostaglandin antagonists such as those disclosed in European Patent Application 11,067 or thromboxane antagonists such as those disclose din U.S. 4,237,160. They may also contain histidine decarboxylase inhibitors such as α-fluoromethylhistidine, described in U.S. 4,325,961. The compounds of formula I may also be advantageously combined with an $H_1$ or $H_2$-receptor antagonist, such as for instance cimetidine, ranitidine, terfenadine, famotidine, temelastine, acrivastine, loratidine, cetrizine, tazifylline, azelastine, aminothiadiazoles disclosed in EP 81102976.8 and like compounds, such as those disclosed in U.S. Patent Nos. 4,283,408; 4,362,736; 4,394,508, and European Patent Application No. 40,696. The pharmaceutical compositions may also contain a $K^+/H^+$ATPase inhibitor such as omeprazole, disclosed in U.S. Patent 4,255,431, and the like.

Thus, "acetic acid derivatives" as defined herein are nonnarcotic analgesics/nonsteroidal antiinflammatory drugs having a free $-CH_2COOH$ group (which optionally can be in the form of a pharmaceutically acceptable salt group, e.g., $-CH_2COO^-Na^+$), typically attached directly to a ring system, preferably to an aromatic or heteroaromatic ring system.

## Claims

1. A compound of the formula (I)

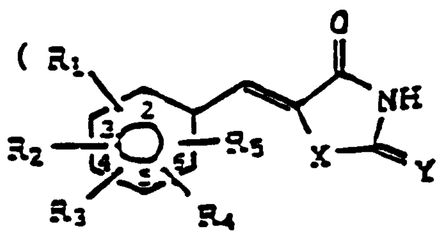

I

and pharmaceutically acceptable salts thereof wherein X and Y are sulfur or oxygen and wherein $R_1$, $R_2$, $R_3$, $R_4$ and $R_5$ are hydrogen or from one to three of $R_1$, $R_2$, $R_3$, $R_4$ and $R_5$ are alkyl or alkoxy of 1 to 6 carbon atoms, hydroxy, halogen, trifluoromethyl, $NO_2$, mercapto, or alkylthio of 1 to 6 carbon atoms or $NR_{10}R_{11}$ wherein $R_{10}$ and $R_{11}$ are independently hydrogen or alkyl of 1 to 6 carbon atoms, however with

proviso 1.) that X and Y may not both be sulfur,

proviso 2.) that when X is sulfur (and Y is oxygen)

a) $R_1$, $R_2$, $R_3$, $R_4$ and $R_5$ may not simultaneously be hydrogen,

b) in case of a monosubstitution of the phenyl ring $R_1$, $R_2$, $R_3$ $R_4$ or $R_5$ is not halogen, hydroxy, nitro, $NR_{10}R_{11}$ or methoxy and

c) in case of a disubstitution of the phenyl ring $R_1$, $R_2$, $R_3$, $R_4$ and $R_5$ is not hydroxy when the second substituent is halogen or hydroxy.

2. A compound of Claim 1 wherein in the formula (I) X is sulfur and Y is oxygen.

3. A compound of Claim 1 wherein in the formula (I) X is oxygen and Y is sulfur.

4. A compound of Claim 1 wherein in of the formula (I) X and Y are both oxygen.

5. A compound of Claim 1 wherein the compound of formula (I) is
5-[(4-hydroxy-3,5-dimethoxyphenyl)methylene]-2,4-thiazolidinedione,
5-[(4-hydroxy-3-methoxyphenyl)methylene]-2,4-thiazolidinedione;
5-[(3,5-bis(1,1-dimethylethyl)-4-hydroxyphenyl)methylene]-2,4-thiazolidinedione and its choline salt;
5-[(4-hydroxy-3-methoxyphenyl)methylene]-2-thioxo-4-oxazolidinoneand its disodium salt;
5-[(4-hydroxy-3,5-dimethoxyphenyl)methylene]-2-thioxo-4-oxazolidinone and its disodium salt;

4-oxazolidinone, 5-[(3-methoxyphenyl)methylene]-2-thioxo- and its disodium salt;
4-oxazolidinone, 5-[(4-methoxyphenyl)methylene]-2-thioxo-;
4-oxazolidinone, 5-[(3,4-dimethoxyphenyl)methylene]-2-thioxo-;
4-oxazolidinone, 5-[(4-hydroxy-3,5-dimethylphenyl) methylene]-2-thioxo;
4-oxazolidinone, 5-[(3-bromo-4-hydroxy-5-methoxyphenyl) methylene]-2-thioxo;
4-oxazolidinone, 2-thioxo-5-[(2,4,5-trimethoxyphenyl) methylene]-;
4-oxazolidinone, 2-thioxo-5-[(3-bromo-4-hydroxyphenyl) methylene]-;
2-thioxo-5-[(3,4,5-trimethoxyphenyl)methylenel]-4-oxazolidinone;
5-[[3,5-bis(1,1-dimethylethyl)-4-hydroxyphenyl]methylene]-2,4-oxazolidinedione;
5-[(4-hydroxy-3,5-dimethoxyphenyl)methylene]-2,4-oxazolidinedione;
5-[(4-hydroxy-3,5-dimethylphenyl)methylene]-2,4-oxazolidinedione;
5-[[3,5-bis(1,1-dimethylethyl)-4-hydroxyphenyl]-methylene]-2-thioxo-oxazolidinone and its choline salt;
5-[[3,5-bis(1,1-dimethylethyl)-4-hydroxyphenyl]-methylene]-2,4-oxazolidinedione;
5-[(4-hydroxy-3,5-dimethoxyphenyl)methylene]-2,4-oxazolidinedione;
5-[(4-hydroxy-3,5-dimethylphenyl)methylene]-2,4-oxazolidinedione;

6. A pharmaceutical composition comprising a compound of claims 1 to 5 and a pharmaceutically acceptable carrier.

7. Use of a compound of the formula (I)

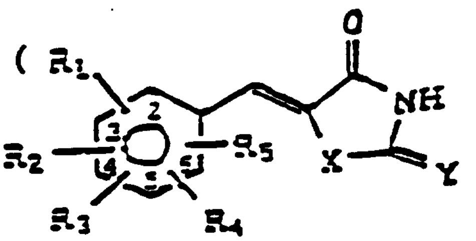

I

and pharmaceutically acceptable salts thereof wherein X and Y are sulfur or oxygen and wherein $R_1$, $R_2$, $R_3$, $R_4$ and $R_5$ are hydrogen or from one to three of $R_1$, $R_2$, $R_3$, $R_4$ and $R_5$ are alkyl or alkoxy of 1 to 6 carbon atoms, hydroxy, halogen, trifluoromethyl, $NO_2$, mercapto, or alkylthio of 1 to 6 carbon atoms or $NR_{10}R_{11}$ wherein $R_{10}$ and $R_{11}$ are independently hydrogen or alkyl of 1 to 6 carbon atoms, however with

proviso 1.) that X and Y may not both be sulfur,

proviso 2.) that when X is sulfur (and Y is oxygen)

a) $R_1$, $R_2$, $R_3$, $R_4$ and $R_5$ may not simultaneously be hydrogen,

b) in case of a monosubstitution of the phenyl ring $R_1$, $R_2$, $R_3$ $R_4$ or $R_5$ is not halogen, hydroxy, nitro, $NR_{10}R_{11}$ or methoxy and

c) in case of a disubstitution of the phenyl ring $R_1$, $R_2$, $R_3$, $R_4$ and $R_5$ is not hydroxy when the second substituent is halogen or hydroxy

for the manufacture of medical therapeutic compositions for treating allergies and inflammations.

8. Use of a compound according to claims 2 to 5 for the manufacture of medical therapeutic compositions for treating allergies and inflammations.

9. A process for preparing a pharmaceutical composition comprising the addition of a compound of the formula (I)

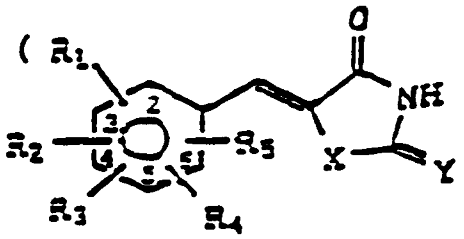

I

or pharmaceutically acceptable salts thereof wherein X and Y are sulfur or oxygen and wherein $R_1$, $R_2$, $R_3$, $R_4$ and $R_5$ are hydrogen or from one to three of $R_1$, $R_2$, $R_3$, $R_4$ and $R_5$ are alkyl or alkoxy of 1 to 6 carbon atoms, hydroxy, halogen, trifluoromethyl, $NO_2$, mercapto, or alxylthio of 1 to 6 carbon atoms or $NR_{10}R_{11}$ wherein $R_{10}$ and $R_{11}$ are independently hydrogen or alkyl of 1 to 6 carbon atoms, however with

proviso 1.) that X and Y may not both be sulfur,

proviso 2.) that when X is sulfur and Y is oxygen

a) $R_1$, $R_2$, $R_3$, $R_4$ and $R_5$ may not simultaneously be hydrogen,

b) in case of a monosubstitution of the phenyl ring $R_1$, $R_2$, $R_3$ $R_4$ or $R_5$ is not halogen, hydroxy, nitro, $NR_{10}R_{11}$ or methoxy and

c) in case of a disubstitution of the phenyl ring $R_1$, $R_2$, $R_3$, $R_4$ and $R_5$ is not hydroxy when the second substituent is halogen or hydroxy.

to a pharmaceutically acceptable carrier.

**10.** A process according to claim 9, wherein in the compound of the formula (I) X is sulfur and Y is oxygen.

**11.** A process according to claim 9, wherein in the compound of formula (I) X is oxygen and Y is sulfur.

**12.** A process of claim 9 wherein in the compound of the formula (I) X and Y are both oxygen.

**13.** A process of claim 9 wherein the compound of formula (I) is
5-[(4-hydroxy-3,5-dimethoxyphenyl)methylene]-2,4-thiazolidinedione,
5-[(4-hydroxy-3-methoxyphenyl)methylene]-2,4-thiazolidinedione;
5-[(3,5-bis(1,1-dimethylethyl)-4-hydroxyphenyl)methylene]-2,4-thiazolidinedione and its choline salt;
5-[(4-hydroxy-3-methoxyphenyl)methylene]-2-thioxo-4-oxazolidinoneand its disodium salt;
5-[(4-hydroxy-3,5-dimethoxyphenyl)methylene]-2-thioxo-4-oxazolidinone and its disodium salt;
4-oxazolidinone, 5-[(3-methoxyphenyl)methylene]-2-thioxo- and its disodium salt;
4-oxazolidinone, 5-[(4-methoxyphenyl)methylene]-2-thioxo-;
4-oxazolidinone, 5-[(3,4-dimethoxyphenyl)methylene]-2-thioxo-;
4-oxazolidinone, 5-[(4-hydroxy-3,5-dimethylphenyl) methylene]-2-thioxo;
4-oxazolidinone, 5-[(3-bromo-4-hydroxy-5-methoxyphenyl) methylene]-2-thioxo;
4-oxazolidinone, 2-thioxo-5-[(2,4,5-trimethoxyphenyl) methylene]-;
4-oxazolidinone, 2-thioxo-5-[(3-bromo-4-hydroxyphenyl) methylene]-;
2-thioxo-5-[(3,4,5-trimethoxyphenyl)methylene]-4-oxazolidinone;
5-[[3,5-bis(1,1-dimethylethyl)-4-hydroxyphenyl]methylene]-2,4-oxazolidinedione;
5-[(4-hydroxy-3,5-dimethoxyphenyl)methylene]-2,4-oxazolidinedione;
5-[(4-hydroxy-3,5-dimethylphenyl)methylene]-2,4-oxazolidinedione;
5-[[3,5-bis(1,1-dimethylethyl)-4-hydroxyphenyl]-methylene]-2-thioxo-oxazolidinone and its choline salt;
5-[[3,5-bis(1,1-dimethylethyl)-4-hydroxyphenyl]-methylene]-2,4-oxazolidinedione;
5-[(4-hydroxy-3,5-dimethoxyphenyl)methylene]-2,4-oxazolidinedione;
5-[(4-hydroxy-3,5-dimethylphenyl)methylene]-2,4-oxazolidinedione;

**14.** A pharmaceutical composition as claimed in claim 6 additionally comprising an effective amount of a second active ingredient that is a nonsteroidal antiinflammatory drug; a peripheral analgesic agent; a cyclooxygenase inhibitor; a leukotriene antagonist; an antihistaminic agent; a prostaglandin antagonist or a thromboxane antagonist.

15. A process of claims 9 to 13 wherein an additional active ingredient that is a nonsteroidal antiinflammatory drug; a peripheral analgesic agent; a cyclooxygenase inhibitor; a leukotriene antagonist; an antihistaminic agent; a prostaglandin antagonist or a thromboxane antagonist is added.

## Patentansprüche

1. Verbindung der Formel (I)

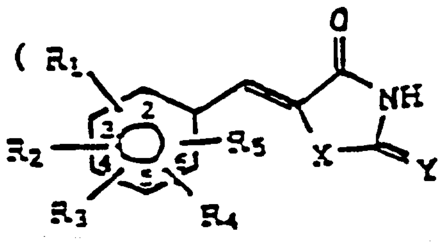

I

worin X und Y für Schwefel oder Sauerstoff stehen und worin $R_1$, $R_2$, $R_3$, $R_4$ und $R_5$ Wasserstoff bedeuten oder 1 bis 3 der Reste $R_1$, $R_2$, $R_3$, $R_4$ und $R_5$ Alkyl oder Alkoxy mit 1 bis 6 Kohlenstoffatom(en), Hydroxy, Halogen, Trifluormethyl, $NO_2$, Mercapto oder Alkylthio mit 1 bis 6 Kohlenstoffatom(en) oder $NR_{10}R_{11}$ mit $R_{10}$ und $R_{11}$ unabhängig voneinander gleich Wasserstoff oder Alkyl mit 1 bis 6 Kohlenstoffatom(en) darstellen, jedoch mit der Ausnahme

1.) daß X und Y nicht gleichzeitig für Schwefel stehen dürfen;

2.) daß, im Falle, daß X für Schwefel steht (und Y Sauerstoff bedeutet)

a) $R_1$, $R_2$, $R_3$, $R_4$ und $R_5$ nicht gleichzeitig Wasserstoff bedeuten dürfen;

b) im Falle einer einfachen Substitution des Phenylrings $R_1$, $R_2$, $R_3$, $R_4$ oder $R_5$ nicht für Halogen, Hydroxy, Nitro, $NR_{10}R_{11}$ oder Methoxy stehen und

c) im Falle einer doppelten Substitution des Phenylrings $R_1$, $R_2$, $R_3$, $R_4$ und $R_5$ nicht für Hydroxy stehen, wenn der zweite Substituent Halogen oder Hydroxy ist,

und deren pharmazeutisch akzeptable Salze.

2. Verbindung nach Anspruch 1, wobei in Formel (I) X für Schwefel und Y für Sauerstoff stehen.

3. Verbindung nach Anspruch 1, wobei in Formel (I) X für Sauerstoff und Y für Schwefel stehen.

4. Verbindung nach Anspruch 1, wobei in Formel (I) X und Y beide für Sauerstoff stehen.

5. Verbindung nach Anspruch 1, wobei es sich bei der Verbindung der Formel (I) um
5-[(4-Hydroxy-3,5-dimethoxyphenyl)methylen]-2,4-thiazolidindion;
5-[(4-Hydroxy-3-methoxyphenyl)methylen]-2,4-thiazolidindion;
5-[(3,5-Bis(1,1-dimethylethyl)-4-hydroxyphenyl)methylen]-2,4-thiazolidindion und sein Cholinsalz;
5-[(4-Hydroxy-3-methoxyphenyl)methylen]-2-thioxo-4-oxazolidinon und sein Dinatriumsalz;
5-[(4-Hydroxy-3,5-dimethoxyphenyl)methylen]-2-thioxo-4-oxazolidinon und sein Dinatriumsalz;
4-Oxazolidinon, 5-[(3-methoxyphenyl)methylen]-2-thioxo- und sein Dinatriumsalz;
4-Oxazolidinon, 5-[(4-methoxyphenyl)methylen]-2-thioxo-;
4-Oxazolidinon, 5-[(3,4-dimethoxyphenyl)methylen]-2-thioxo-;
4-Oxazolidinon, 5-[(4-hydroxy-3,5-dimethylphenyl)methylen]-2-thioxo;
4-Oxazolidinon, 5-[(3-brom-4-hydroxy-5-methoxyphenyl)methylen]-2-thioxo;
4-Oxazolidinon, 2-thioxo-5-[(2,4,5-trimethoxyphenyl)methylen]-;
4-Oxazolidinon, 2-thioxo-5-[(3-brom-4-hydroxyphenyl)methylen]-;
2-Thioxo-5-[(3,4,5-trimethoxyphenyl)methylen]-4-oxazolidinon;
5-[(3,5-Bis(1,1-dimethylethyl)-4-hydroxyphenyl]methylen]-2,4-oxazolidindion;
5-[(4-Hydroxy-3,5-dimethoxyphenyl)methylen]-2,4-oxazolidindion;
5-[(4-Hydroxy-3,5-dimethylphenyl)methylen]-2,4-oxazolidindion;
5-[(3,5-Bis(1,1-dimethylethyl)-4-hydroxyphenyl]-methylen]-2-thioxooxazolidinon und sein Cholinsalz;
5-[(3,5-Bis(1,1-dimethylethyl)-4-hydroxyphenyl]-methylen]-2,4-oxazolidindion;
5-[(4-Hydroxy-3,5-dimethoxyphenyl)methylen]-2,4-oxazolidindion;

5-[(4-Hydroxy-3,5-dimethylphenyl)methylen]-2,4-oxazolidindion; handelt.

**6.** Arzneimittelzubereitung, umfassend eine Verbindung nach Ansprüchen 1 bis 5 und einen pharmazeutisch akzeptablen Träger.

**7.** Verwendung einer Verbindung der Formel (I)

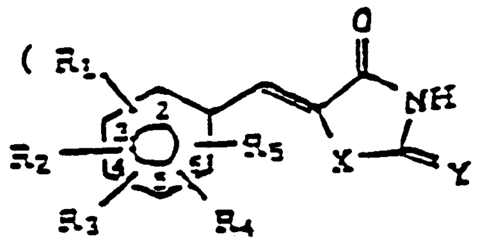

I

worin X und Y für Schwefel oder Sauerstoff stehen und worin $R_1$, $R_2$, $R_3$, $R_4$ und $R_5$ Wasserstoff bedeuten oder 1 bis 3 der Reste $R_1$, $R_2$, $R_3$, $R_4$ und $R_5$ Alkyl oder Alkoxy mit 1 bis 6 Kohlenstoffatom(en), Hydroxy, Halogen, Trifluormethyl, $NO_2$, Mercapto oder Alkylthio mit 1 bis 6 Kohlenstoffatom(en) oder $NR_{10}R_{11}$ mit $R_{10}$ und $R_{11}$ unabhängig voneinander gleich Wasserstoff oder Alkyl mit 1 bis 6 Kohlenstoffatom(en) darstellen, jedoch mit der Ausnahme
1.) daß X und Y nicht gleichzeitig für Schwefel stehen dürfen;
2.) daß, im Falle, daß X für Schwefel steht (und Y Sauerstoff bedeutet)
a) $R_1$, $R_2$, $R_3$, $R_4$ und $R_5$ nicht gleichzeitig Wasserstoff bedeuten dürfen;
b) im Falle einer einfachen Substitution des Phenylrings $R_1$, $R_2$, $R_3$, $R_4$ oder $R_5$ nicht für Halogen, Hydroxy, Nitro, $NR_{10}R_{11}$ oder Methoxy stehen und
c) im Falle einer doppelten Substitution des Phenylrings $R_1$, $R_2$, $R_3$, $R_4$ und $R_5$ nicht für Hydroxy stehen, wenn der zweite Substituent Halogen oder Hydroxy ist,
und von pharmazeutisch akzeptablen Salzen derselben für die Herstellung therapeutischer Arzneimittelzubereitungen zur Behandlung von Allergien und Entzündungen.

**8.** Verwendung einer Verbindung nach Ansprüchen 2 bis 5 für die Herstellung therapeutischer Arzneimittelzubereitungen zur Behandlung von Allergien und Entzündungen.

**9.** Verfahren zur Herstellung einer Arzneimittelzubereitung durch Zusatz einer Verbindung der Formel (I)

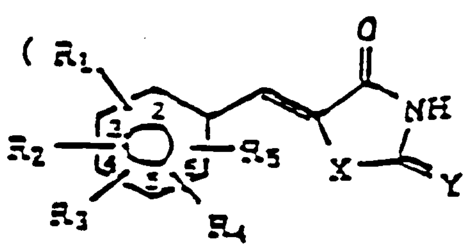

I

worin X und Y für Schwefel oder Sauerstoff stehen und worin $R_1$, $R_2$, $R_3$, $R_4$ und $R_5$ Wasserstoff bedeuten oder 1 bis 3 der Reste $R_1$, $R_2$, $R_3$, $R_4$ und $R_5$ Alkyl oder Alkoxy mit 1 bis 6 Kohlenstoffatom(en), Hydroxy, Halogen, Trifluormethyl, $NO_2$, Mercapto oder Alkylthio mit 1 bis 6 Kohlenstoffatom(en) oder $NR_{10}R_{11}$ mit $R_{10}$ und $R_{11}$ unabhängig voneinander gleich Wasserstoff oder Alkyl mit 1 bis 6 Kohlenstoffatom(en) darstellen, jedoch mit der Ausnahme
1.) daß X und Y nicht gleichzeitig für Schwefel stehen dürfen;
2.) daß, im Falle, daß X für Schwefel steht (und Y Sauerstoff bedeutet)
a) $R_1$, $R_2$, $R_3$, $R_4$ und $R_5$ nicht gleichzeitig Wasserstoff bedeuten dürfen;

b) im Falle einer einfachen Substitution des Phenylrings $R_1$, $R_2$, $R_3$, $R_4$ oder $R_5$ nicht für Halogen, Hydroxy, Nitro, $NR_{10}R_{11}$ oder Methoxy stehen und

c) im Falle einer doppelten Substitution des Phenylrings $R_1$, $R_2$, $R_3$, $R_4$ und $R_5$ nicht für Hydroxy stehen, wenn der zweite Substituent Halogen oder Hydroxy ist,

oder von pharmazeutisch akzeptable Salzen derselben zu einem pharmazeutisch akzeptablen Träger.

**10.** Verfahren nach Anspruch 9, wobei in Formel (I) X für Schwefel und Y für Sauerstoff stehen.

**11.** Verfahren nach Anspruch 9, wobei in Formel (I) X für Sauerstoff und Y für Schwefel stehen.

**12.** Verfahrung nach Anspruch 9, wobei in Formel (I) X und Y beide für Sauerstoff stehen.

**13.** Verfahren nach Anspruch 9, wobei es sich bei der Verbindung der Formel (I) um
5-[(4-Hydroxy-3,5-dimethoxyphenyl)methylen]-2,4-thiazolidindion;
5-[(4-Hydroxy-3-methoxyphenyl)methylen]-2,4-thiazolidindion;
5-[(3,5-Bis(1,1-dimethylethyl)-4-hydroxyphenyl)methylen]-2,4-thiazolidindion und sein Cholinsalz;
5-[(4-Hydroxy-3-methoxyphenyl)methylen]-2-thioxo-4-oxazolidinon und sein Dinatriumsalz;
5-[(4-Hydroxy-3,5-dimethoxyphenyl)methylen]-2-thioxo-4-oxazolidinon und sein Dinatriumsalz;
4-Oxazolidinon, 5-[(3-methoxyphenyl)methylen]-2-thioxo- und sein Dinatriumsalz;
4-Oxazolidinon, 5-[(4-methoxyphenyl)methylen]-2-thioxo-;
4-Oxazolidinon, 5-[(3,4-dimethoxyphenyl)methylen]-2-thioxo-;
4-Oxazolidinon, 5-[(4-hydroxy-3,5-dimethylphenyl)methylen]-2-thioxo;
4-Oxazolidinon, 5-[(3-brom-4-hydroxy-5-methoxyphenyl)methylen]-2-thioxo;
4-Oxazolidinon, 2-thioxo-5-[(2,4,5-trimethoxyphenyl)methylen]-;
4-Oxazolidinon, 2-thioxo-5-[(3-brom-4-hydroxyphenyl)methylen]-;
2-Thioxo-5-[(3,4,5-trimethoxyphenyl)methylen]-4-oxazolidinon;
5-[(3,5-Bis(1,1-dimethylethyl)-4-hydroxyphenyl]methylen]-2,4-oxazolidindion;
5-[(4-Hydroxy-3,5-dimethoxyphenyl)methylen]-2,4-oxazolidindion;
5-[(4-Hydroxy-3,5-dimethylphenyl)methylen]-2,4-oxazolidindion;
5-[(3,5-Bis(1,1-dimethylethyl)-4-hydroxyphenyl]-methylen]-2-thioxooxazolidinon und sein Cholinsalz;
5-[(3,5-Bis(1,1-dimethylethyl)-4-hydroxyphenyl]-methylen]-2,4-oxazolidindion;
5-[(4-Hydroxy-3,5-dimethoxyphenyl)methylen]-2,4-oxazolidindion;
5-[(4-Hydroxy-3,5-dimethylphenyl)methylen]-2,4-oxazolidindion;
handelt.

**14.** Arzneimittelzubereitung nach Anspruch 6, zusätzlich enthaltend eine wirksame Menge eines zweiten aktiven Bestandteils, bei dem es sich um ein entzündungshemmendes oder -widriges nicht-Steroid-Arzneimittel, ein periferes Analgeticum, einen Cyclooxygenaseinhibitor, einen Leukotrienantagonisten, ein Antihistaminikum, einen Prostaglandinantagonisten oder einen Thromboxanantagonisten handelt.

**15.** Verfahren nach Ansprüchen 9 bis 13, bei welchem ein zusätzlicher aktiver Bestandteil, bei dem es sich um ein entzündungshemmendes oder -widriges nicht-Steroid-Arzneimittel, ein periferes Analgeticum, einen Cyclooxygenaseinhibitor, einen Leukotrienantagonisten, ein Antihistaminikum, einen Prostaglandinantagonisten oder einen Thromboxanantagonisten handelt, zugesetzt wird.

## Revendications

**1.** Un composé de formule (I):

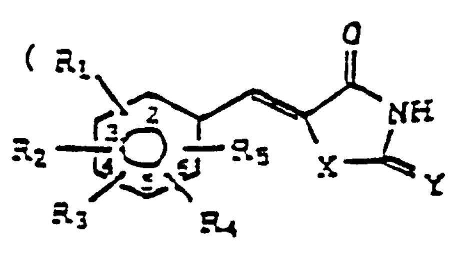

I

et ses sels pharmaceutiquement acceptables en dérivant,
dans laquelle:

X et Y sont des atomes de soufre et d'oxygène, et
$R_1$, $R_2$, $R_3$, $R_4$ et $R_5$ sont de l'hydrogène, ou de un à trois
$R_1$, $R_2$, $R_3$, $R_4$ et $R_5$ sont des groupes alkyle ou alcoxy contenant 1 à 6 atomes de carbone, hydroxy, halogène, trifluorométhyle, $NO_2$, mercapto ou alkylthio contenant 1 à 6 atomes de carbone, ou $NR_{10}R_{11}$,

dans lequel:

$R_{10}$ et $R_{11}$ sont indépendamment de l'hydrogène ou un groupe alkyle de 1 à 6 atomes de carbone,

avec cependant la condition que:

1) X et Y ne sont pas tout deux du soufre,
2) lorsque X est du soufre (et Y de l'oxygène):
   a) $R_1$, $R_2$, $R_3$, $R_4$ et $R_5$ ne peuvent pas être simultanément de l'hydrogène,
   b) dans le cas d'une monosubstitution du cycle phényle, $R_1$, $R_2$, $R_3$, $R_4$ ou $R_5$ n'est pas un halogène, hydroxy, nitro, $NR_{10}R_{11}$ ou méthoxy, et
   c) dans le cas d'une disubstitution du cycle phényle, $R_1$, $R_2$, $R_3$, $R_4$ et $R_5$ ne sont pas hydroxy lorsque le second substituant est halogène ou hydroxy.

**2.** Un composé selon la revendication 1, dans lequel dans la formule (I), X est du soufre et Y de l'oxygène.

**3.** Un composé selon la revendication 1, dans lequel dans la formule (I), X est de l'oxygène et Y du soufre.

**4.** Un composé selon la revendication 1, dans lequel dans la formule (I), X et Y sont tout deux de l'oxygène.

**5.** Un composé selon la revendication 1, dans lequel dans le composé de formule (I) est:

5-[(4-hydroxy-3,5-diméthoxyphényl)méthylène]-2,4-thiazolidinedione,
5-[(4-hydroxy-méthoxyphényl)méthylène]-2,4-thiazolidinedione,
5-[(3,5-bis-(1,1-diméthyléthyl)-4-hydroxyphényl)méthylène]-2,4-thiazolidinedione et ses sels de choline,
5-[(4-hydroxy-3-méthoxyphényl)méthylène]-2-thioxo-4-oxazolidinone, et son sel disodique,
5-[(4-hydroxy-3,5-diméthoxyphényl)méthylène]-2-thioxo-4-oxazolidinone, et son sel disodique,
5-[(3-méthoxyphényl)méthylène]-2-thioxo-4-oxazolidinone, et son sel disodique,
5-[(4-méthoxyphényl)méthylène]-2-thioxo-4-oxazolidinone,
5-[(3,4-diméthoxyphényl)méthylène]-2-thioxo-4-oxazolidinone,
5-[(4-hydroxy-3,5-diméthylphényl)méthylène]-2-thioxo-4-oxazolidinone,
5-[(3-bromo-4-hydroxy-5-méthoxyphényl)méthylène]-2-thioxo-4-oxazolidinone,
2-thioxo-5-[(2,4,5-triméthoxyphényl)méthylène-4-oxazolidinone],
2-thioxo-5-[(3-bromo-4-hydroxyphényl)méthylène-4-oxazolidinone],
2-thioxo-5-[(3,4,5-triméthoxyphényl)méthylène]-4-oxazolidinone,
5-[[(3,5-bis-(1,1-diméthyléthyl)-4-hydroxyphényl]méthylène]-2,4-oxazolidinedione,
5-[(4-hydroxy-3,5-diméthoxyphényl)méthylène]-2,4-oxazolidinedione,
5-[(4-hydroxy-3,5-diméthylphényl)méthylène]-2,4-oxazolidinedione,
5-[[3,5-bis-(1,1-diméthyléthyl)-4-hydroxyphényl]méthylène]-2-thioxo-oxazolidinone et son sel de choline,
5-[[3,5-bis-(1,1-diméthyléthyl)-4-hydroxyphényl]méthylène]-2,4-oxazolidinedione,
5-[(4-hydroxy-3,5-diméthoxyphényl)méthylène]-2,4-oxazolidinedione, et
5-[(4-hydroxy-3,5-diméthylphényl)méthylène]-2,4-oxazolidinedione.

**6.** Une composition pharmaceutique comprenant un composé selon les revendications 1 à 5 et un support pharmaceutiquement 8acceptable.

**7.** Utilisation d'un composé de formule (I)

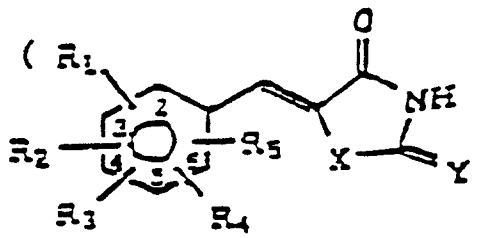

I

et de ses sels pharmaceutiquement acceptables en dérivant,
dans laquelle:

| | |
|---|---|
| X et Y | sont des atomes de soufre et d'oxygène, et |
| $R_1$, $R_2$, $R_3$, $R_4$ et $R_5$ | sont de l'hydrogène, ou de un à trois $R_1$, $R_2$, $R_3$, $R_4$ et $R_5$ sont des groupes alkyle ou alcoxy contenant 1 à 6 atomes de carbone, hydroxy, halogène, trifluorométhyle, $NO_2$, mercapto ou alkylthio contenant 1 à 6 atomes de carbone, ou $NR_{10}R_{11}$, dans lequel: |
| $R_{10}$ et $R_{11}$ | sont indépendamment de l'hydrogène ou un groupe alkyle de 1 à 6 atomes de carbone, avec cependant la condition que: |

1) X et Y ne sont pas tout deux du soufre,
2) lorsque X est du soufre (et Y de l'oxygène):
a) $R_1$, $R_2$, $R_3$, $R_4$ et $R_5$ ne peuvent pas être simultanément de l'hydrogène,
b) dans le cas d'une monosubstitution du cycle phényle, $R_1$, $R_2$, $R_3$, $R_4$ ou $R_5$ n'est pas un halogène, hydroxy, nitro, $NR_{10}R_{11}$ ou méthoxy, et
c) dans le cas d'une disubstitution du cycle phényle, $R_1$, $R_2$, $R_3$, $R_4$ et $R_5$ ne sont pas hydroxy lorsque le second substituant est halogène ou hydroxy,

pour la fabrication de compositions thérapeutiques médicales pour le traitement des allergies et des inflammations.

**8.** Utilisation d'un composé selon les revendications 2 à 5 pour la fabrication de compositions thérapeutiques médicales pour le traitement des allergies et des inflammations.

**9.** Un procédé de préparation d'une composition pharmaceutique comprenant l'addition d'un composé de formule (I):

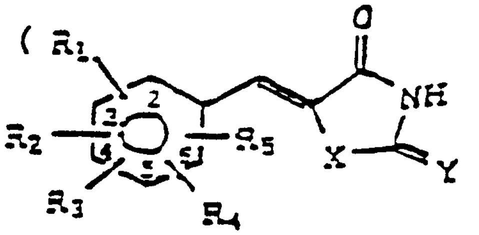

I

et de ses sels pharmaceutiquement acceptables en dérivant,
dans laquelle:

| | |
|---|---|
| X et Y | sont des atomes de soufre et d'oxygène, et |
| $R_1$, $R_2$, $R_3$, $R_4$ et $R_5$ | sont de l'hydrogène, ou de un à trois $R_1$, $R_2$, $R_3$, $R_4$ et $R_5$ sont des groupes alkyle ou alcoxy de 1 à 6 atomes de carbone, hydroxy, halogène, trifluorométhyle, $NO_2$, mercapto ou alkylthio de 1 à 6 atomes de carbone, ou $NR_{10}R_{11}$, |

dans lequel:

$R_{10}$ et $R_{11}$ sont indépendamment de l'hydrogène ou un groupe alkyle de 1 à 6 atomes de carbone,

avec cependant la condition que:

1) X et Y ne sont pas tout deux du soufre,

2) lorsque X est du soufre (et Y de l'oxygène):

a) $R_1$, $R_2$, $R_3$, $R_4$ et $R_5$ ne peuvent pas être simultanément de l'hydrogène,

b) dans le cas d'une monosubstitution du cycle phényle, $R_1$, $R_2$, $R_3$, $R_4$ ou $R_5$ n'est pas un halogène, hydroxy, nitro, $NR_{10}R_{11}$ ou méthoxy, et

c) dans le cas d'une disubstitution du cycle phényle, $R_1$, $R_2$, $R_3$, $R_4$ et $R_5$ ne sont pas hydroxy lorsque le second substituant est halogène ou hydroxy,

à un support pharmaceutiquement acceptable.

**10.** Un procédé selon la revendication 9, dans lequel, dans le composé formule (I), X est du soufre et Y de l'oxygène.

**11.** Un procédé selon la revendication 9, dans lequel, dans le composé de formule (I), X est de l'oxygène et Y du soufre.

**12.** Un procédé selon la revendication 9, dans lequel, dans le composé de formule (I), X et Y sont tout deux de l'oxygène.

**13.** Un procédé selon la revendication 9, dans lequel le composé de formule (I) est:

5-[(4-hydroxy-3,5-diméthoxyphényl)méthylène]-2,4-thiazolidinedione,
5-[(4-hydroxy-3,5-méthoxyphényl)méthylène]-2,4-thiazolidinedione,
5-[(3,5-bis-(1,1-diméthyléthyl)-4-hydroxyphényl)méthylène]-2,4-thiazolidinedione et ses sels de choline,
5-[(4-hydroxy-3-méthoxyphényl)méthylène]-2-thioxo-4-oxazolidinone, et son sel disodique,
5-[(4-hydroxy-3,5-diméthoxyphényl)méthylène]-2-thioxo-4-oxazolidinone, et son sel disodique,
5-[(3-méthoxyphényl)méthylène]-2-thioxo-4-oxazolidinone et son sel disodique,
5-[(4-méthoxyphényl)méthylène]-2-thioxo-4-oxazolidinone,
5-[(3,4-diméthoxyphényl)méthylène]-2-thioxo-4-oxazolidinone,
5-[(4-hydroxy-3,5-diméthylphényl)méthylène]-2-thioxo-4-oxazolidinone,
5-[(3-bromo-4-hydroxy-5-méthoxyphényl)méthylène]-2-thioxo-4-oxazolidinone,
2-thioxo-5-[(2,4,5-triméthoxyphényl)méthylène]-4-oxazolidinone,
2-thioxo-5-[(3-bromo-4-hydroxyphényl)méthylène]-4-oxazolidinone,
2-thioxo-5-[(3,4,5-triméthoxyphényl)méthylène]-4-oxazolidinone,
5-[(3,5-bis-(1,1-diméthoxyphényl)méthylène]-2,4-oxazolidinedione,
5-[(4-hydroxy-3,5-diméthylphényl)méthylène]-2,4-oxazolidinedione,
5-[(4-hydroxy-3,5-diméthylphényl)méthylène]-2,4-oxazolidinedione,
5-[[3,5-bis-(1,1-diméthyléthyl)-4-hydroxyphényl]méthylène]-2-thioxo-oxazolidinone et son sel de choline,
5-[[3,5-bis-(1,1-diméthyléthyl)-4-hydroxyphényl]méthylène]-2,4-oxazolidinedione,
5-[(4-hydroxy-3,5-diméthoxyphényl)méthylène]-2,4-oxazolidinedione, et
5-[(4-hydroxy-3,5-diméthylphényl)méthylène]-2,4-oxazolidinedione.

**14.** Une composition pharmaceutique selon la revendication 6, qui comprend de plus une quantité efficace d'un second ingrédient actif consistant en un médicament anti-inflammatoire non-stéroïdien, un agent analgésique périphérique, un inhibiteur de la cyclooxygénase, un antagoniste de leukotriène, un agent antihistaminique, un antagoniste de la prostaglandine ou un antagoniste de la thromboxane.

**15.** Un procédé selon la revendication 9, dans lequel on ajoute aussi un ingrédient actif supplémentaire qui est un médicament non-stéroïdien, anti-inflammatoire, un agent analgésique périphérique, un inhibiteur de la cyclooxygénase, un antagoniste de leukotriène, un agent antihistaminique, un antagoniste de la prostaglandine un antagoniste de la thromboxane.